# EUROPEAN PATENT SPECIFICATION

(11) **EP 2 570 124 B1**
(45) Date of publication and mention of the grant of the patent: **02.11.2016**
(21) Application number: 11743251.8
(22) Date of filing: 13.05.2011
(51) Int. Cl.: A61K 31/351, A61K 31/7004, A61K 36/63, A61K 9/06, A61K 9/08, A61P 17/02, A61K 47/10, A61K 47/32, A61K 31/7048

(54) **OLEUROPEIN COMPOSITIONS FOR HEALING WOUNDS AND ULCERS IN ELDERLY PEOPLE AND/OR DIABETICS**
OLEUROPEIN ZUSAMMENSETZUNGEN ZUR BEHANDLUNG VON WUNDEN UND GESCHWÜREN BEI ÄLTEREN MENSCHEN UND/ODER DIABETIKERN
COMPOSITIONS D'OLEUROPÉINE POUR LA CICATRISATION DE PLAIES ET D'ULCÈRES CHEZ DES PERSONNES ÂGÉES ET/OU DIABÉTIQUES

(30) Priority: 14.05.2010 WO PCT/ES2010/070327
(43) Date of publication of application: 20.03.2013
(73) Proprietor: Sanidad Y Residencias 21, S.A., 14006 Córdoba (ES); Servicio Andaluz de Salud, 41001 Sevilla (ES); Quesper, R&D, S.L., 14004 Madrid (ES)
(72) Inventor: QUESADA GÓMEZ, José Manuel, E-14004 Córdoba (ES); SANTIAGO MORA, Raquel María, E-14010 Córdoba (ES); CASADO DÍAZ, Antonio, E-14650 Bujalance (Córdoba) (ES)
(74) Representative: Carlos Hernando, Borja
(86) International application number: PCT/ES2011/070343
(87) International publication number: WO 2011/141611

(56) References cited:
- WO-A1-2007/006484
- WO-A1-2007/051829
- WO-A1-2010/070183
- WO-A1-2010/130869
- WO-A2-2004/096119
- WO-A2-2005/063195
- WO-A2-2006/137100
- DE-U1- 20 301 401
- MX-A- 2007 016 417
- US-A1- 2004 097 428
- US-A1- 2004 101 507
- US-A1- 2006 287 234
- GALIANO ROBERT D ET AL: "Topical vascular endothelial growth factor accelerates diabetic wound healing through increased angiogenesis and by mobilizing and recruiting bone marrow-derived cells.", THE AMERICAN JOURNAL OF PATHOLOGY JUN 2004, vol. 164, no. 6, June 2004 (2004-06), pages 1935-1947, ISSN: 0002-9440

## Description

### Technical Field of the Invention

The present invention relates to a new use of oleuropein in the treatment of healing wounds and ulcers in persons of advanced age or the elderly and/or diabetics.

Particularly, the present invention specifically relates to compositions containing oleuropein as the only active ingredient for use in the treatment of healing wounds and ulcers in
persons of advanced age, the elderly and/or diabetics, selected from the group consisting of: diabetic foot, pressure ulcer and venous ulcers.

### Background of the Invention

Oleuropein is mainly, but not exclusively, obtained from the leaves of the *Olea europea* olive tree, native of the Mediterranean area where both the fruit and the oil extracted therefrom are the main ingredients of what is called the "Mediterranean diet".

Oleuropein is the main phenolic component of the pulp of green olives (of the *Olea europea* variety which the glycoside is named after) and is found in the virgin olive oil to which it gives its bitter taste. Olive leaves also contain oleuropein as the main component. The active ingredient (a.i.) used in the present invention, i.e., oleuropein, was obtained from olive leaf extracts (European Pharmacopoeia 6.4: minimum oleuropein content of 16%) and subsequent isolation and purification.

Olive extract is not only healthy but it is the basis for building a nontoxic immune system¹. Clinical evidence has demonstrated that the olive leaf extracts reduce blood pressure^{2,3,4,5}. It has also been published that a liquid extract made directly from fresh olive leaves has an antioxidant capacity of almost twice that made from a green tea extract and 400% higher than vitamin C⁶. Non-patent literature articles describe oleuropein as having various pharmacological properties, such as for example, antioxidant⁷, anti-inflammatory⁸, anti-carcinogenic^{9,10}, anitimicrobial¹¹ and antiviral¹² properties. Oleuropein has further demonstrated to be cardioprotective against cardiotoxicity of doxorubicin¹³ and it has been seen to show anti-ischemic and hypolipidemic activities¹⁴.

With respect to patent documents, US 2004/0097428 A1 relates to methods for inhibiting cancer and other diseases leading to scar formation which comprises administering oleuropein and the products of its hydrolysis in therapeutically effective amounts (claim 12 mentioned more than one hundred types of cancer).

US 2002/0110600 A1 relates to a composition for relief of symptoms of cold and influenza comprising 80-88% of an olive leaf extract containing oleuropein. KR 20070065955 relates to a toothpaste comprising microcapsules which contain functional ingredients to provide direct contact of the oleuropein with the oral cavity to inhibit bacteria, fungi and to improve the inner mucosal skin of the mouth.

Document ES 2 274 721 A1 relates to the use of oleuropein for producing a medicinal product for the preventive and/or therapeutic treatment of a condition related to peripheral vascular disease, particularly venous insufficiency where the disorder are hemorrhoids or varicose veins.

Document US2004/097428 relates to the use
of oleuropein for inhibiting cancer and scar formation.

Document US 2003/0004117 A1 relates to methods for inhibiting angiogenesis by means of using a composition in a plant extract form comprising oleuropein.

Document WO2010/070183 A1, of the same authors as the present application, relates to pharmaceutical compositions comprising oleuropein and olive leaf extracts to induce angiogenesis and vasculogenesis.

Document MX 2007016417 A relates to a composition comprising 1-25% strain L *allium* extract as the essential ingredient in addition to a mixture of polyethylene glycol (PEG) comprising 15-50% PEG 4000 and 5-30% PEG 300, an alcohol (preferably 15-50% ethanol or ethanol:water at a ratio of 50:10%) and a plant oil (claims 1-2, 4-5) preferably olive oil at 5-20% (claim 3); the preparation of the composition (claim 6) and the use thereof for the prevention or the treatment of diabetic foot, mainly infected wounds and external ulcerations of the skin (claim 7). However, this document does not consider olive oil and/or its components as the essential ingredients of the invention since it is only incorporated to obtain the formulation in liquid composition form. Other formulations described in the document, such as the ointment, do not incorporate said component, using high and low molecular weight polyethylene glycol mixtures to obtain the desired ointment formulation.

Thus, it can be concluded that oleuropein is known for the treatment of different diseases and as the modulator of various physiological processes. However, attempts to cure certain diseases affecting a specific group of persons, such as persons of advanced age, the elderly and/or diabetics, and more specifically diseases of said groups, such as diabetic foot, pressure ulcer and venous ulcers, with oleuropein have been unknown up until now.

### Object of the Invention

The object of the present invention is therefore pharmaceutical compositions containing oleuropein as the sole active ingredient for use in the treatment of healing wounds and ulcers in persons of advanced
age or the elderly and/or diabetics.

In a particular embodiment of the present invention, the compositions contain oleuropein with a degree of purity of at least 30%, preferably between 41.5% and 100%, specifically 41.5% and 80%, as the only active ingredient for use in the treatment of healing wounds and ulcers in a specific group of patients such as persons of advanced age, the elderly and/or diabetics, selected from the group consisting of: diabetic foot, pressure ulcer and venous ulcers.

The compositions of the present invention therefore contain oleuropein with a determined degree of purity as the only active ingredient in the formulation, as well as the corresponding pharmaceutically acceptable carriers commonly used in the pharmaceutical industry.

For the purpose of the present invention, persons of advanced age and/or the elderly are considered a specific patient group characterized by presenting determined symptoms and/or physical functional condition and age.

The symptoms and/or physical functional condition typical of the group of patients selected for the purpose of the present invention, persons of advanced age, are generally a combination of different factors, among others, chronic disease, vascular insufficiency, chronic venous insufficiency, growth factor deficiency, lack of tissue regeneration, diabetes, anemia, hypoproteinemia, obesity, neurological defects, nutritional deficiencies, immobility, etc.

The typical age of the treatment group referred to in the present invention is above 55 years of age, preferably those persons older than 65 years of age, more preferably, persons older than 70 years of age.

The following are therefore the objects of the present invention:
A pharmaceutical composition containing oleuropein as the only active ingredient for use in the treatment of healing wounds and ulcers in persons of advanced age or the
   elderly and/or diabetics, selected from the group consisting of: diabetic foot, pressure ulcer and venous ulcer.

A pharmaceutical composition comprising oleuropein for use in the method of the invention, characterized in that the
oleuropein is present at a concentration of 10⁻¹M to 10⁻¹¹M, preferably at a concentration of 10⁻² to 10⁻⁹M, more preferably at a concentration of 10⁻².

A pharmaceutical composition containing oleuropein for use in the method of the invention, characterized in that the oleuropein present in the composition has a degree of purity of at least 30%, preferably between 41.5% and 100%, specifically 41.5% and 80%.

A pharmaceutical composition containing oleuropein for use in the method of the invention, characterized in that it is presented in gel, cream (o/w emulsion), or aqueous solution form and additionally comprises the corresponding pharmaceutically acceptable carriers.

The pharmaceutical compositions of the present invention for use in the method of the invention, in an
application regimen at 12 hours, 24 hours and 48 hours.

### Description of the Drawings

Figure 1: Venous ulcer. Phase I: Percentage of daily reduction of the ulcerated area at a concentration of 10⁻²M, gel, cream and aqueous solution.
Figure 2: Venous ulcer. Phase II: Percentage of progression of healed surface at a concentration of 10⁻¹⁰M.
Figure 3: Venous ulcer. Phase III: Percentage of progression of healed surface at a concentration of 10⁻¹M.
Figure 4: Venous ulcer. Phase IV: Average percentage of progression of healed surface at concentrations of 10⁻¹M - 10⁻¹¹M.
Figure 5: Venous ulcer. Phase IV: Average percentage of daily reduction of ulcer at concentrations of 10⁻¹⁰M - 10⁻¹¹M.
Figure 6: Venous ulcer. Phase V: Percentage of daily reduction of the ulcerated area at a concentration of 10⁻²M and application regimens at 12 hours, 24 hours, and 48 hours.
Figure 7: Venous ulcer. Phase VI: Percentage of daily reduction of the ulcerated area. Oleuropein with a degree of purity of 41.5% vs. 80%.
Figure 8: Diabetic foot: Percentage of daily reduction of the ulcerated area at concentrations of 10⁻²M and 10⁻⁹M and application regimens at 12 hours and 24 hours.
Figure 9: Pressure ulcer: Healed surface at the end of the study at concentrations of 10⁻²M, 10⁻⁷M, 10⁻⁹M and application regimens at 12 hours, 24 hours and 48 hours.

### Detailed Description of the Invention

As discussed above, the symptom and/or physical functional condition determining the diseases to be treated in the present invention in persons of advanced age is generally a combination of different factors, including among others chronic disease, vascular insufficiency, chronic venous insufficiency, growth factor deficiency, lack of tissue regeneration, anemia, diabetes, hypoproteinemia, obesity, neurological defects, nutritional deficiencies, immobility, etc.

An explanation of the factors causing the generation and development of each of the specific diseases to be treated will be discussed below.

According to the "International Working Group on the Diabetic Foot", diabetic foot is an infection, ulceration or destruction of the deep tissues related with neurological alterations and different degrees of peripheral vascular disease in the lower extremities affecting patients with diabetes mellitus^{15,16}. It is important to emphasize that "diabetic foot" must not be confused with the foot of a diabetic person, since not all diabetics develop this complication that depends largely on the control that the diabetic has over the disease, the intrinsic factors and environmental factors associated with the patient, and ultimately on the stage of progression of the base pathology.

When diabetic foot is said to have a "neuropathic etiopathogenic basis", it means that the primary cause leading to one suffering from diabetic foot is in the progressive damage caused by diabetes on the nerves, known as neuropathy. The nerves are responsible for reporting on different stimuli (sensory nerves) and for controlling the muscles (effector nerves)¹⁷. In diabetics, nerve involvement causes the patient to lose sensitivity, especially pain and heat sensitivity, and it causes the muscles to atrophy, favoring the onset of foot deformities since the muscles are inserted into the bones, mobilize them and stabilize the bone structure¹⁸.

The fact that a person looses sensitivity in the foot implies that if a wound, excessive rubbing, overpressure of a determined point or exposure to excessive heat or cold sources occurs, they will not be felt.¹⁹ Since pain is a defense mechanism of an organism which encourages one to take measures to protect against aggressive factors, diabetics may suffer from wounds and not realize it. Furthermore, the loss of muscle control favors the onset of deformities and these may, at the same time, favors rubbing, changes in the distribution of the foot supports during walking and predisposing aggressions to the determined points of the foot which, if not taken care of in time, may be fatal.^{20,21,22}

Ischemia or tissue damage derived from arterial insufficiency is common in diabetics as a consequence of the damage suffered by the blood vessels of these patients due to the disease. The function of arteries is to provide nutrients and oxygen to cells so that they function properly. The foot is a risk area compromised by its distance to the heart and if the damage suffered by the blood vessels is added to this, it can be seen that arterial circulation of the foot is greatly reduced.

Regarding the factors triggering diabetic foot²³, it has been found that starting from weak dry skin, blood flow insufficiency added to a lack of sensitivity of the patient, a traumatism, such as a hit, a sharp or cutting element²⁴, a small stone which gets into the shoes or, to take an obvious example, chafing produced by tight footwear²⁵, may cause the start of the disease.

Thus a wound is formed which the diabetic is not even aware of at first due to the loss of sensitivity. If left untreated, this wound is an open door for infection-causing microorganisms. Since the inflammatory response is lowered, the pathogens do not meet sufficient resistance, colonize the area, the tissue is destroyed and has trouble healing. Ulceration is therefore produced. Ulcers are wounds characterized by a loss of substance and especially by their little tendency to heal²⁶.

Ulcers require all the podiatric and medical care within the patient's reach because if not heal spontaneously, ulcers tend to deteriorate, becoming gangrenous in many cases, forcing partial amputations or even complete amputations of the affected areas. It is estimated that complications derived from diabetes are the main cause of non-traumatic amputation worldwide²⁷. Diabetic foot ulcers require a multidisciplinary diagnosis and treatment, generally carried out by diabetes specialists and surgeons. The treatment known in the state of the art consists of suitable bandages, antibiotics (against staphylococcus, streptococcus and anaerobic strains), debridement and arterial revascularization.

Specialists are studying the role of nitric oxide in healing a diabetic foot wound²⁸. Nitric oxide, which is already used in the state of the art, is a powerful vasodilator which aids in carrying nutrients to oxygen-deficient wounds. A study was conducted in 2004 which concluded that for people with diabetic foot, hyperbaric oxygen therapy (medical use of the oxygen at a pressure higher than the atmospheric pressure) may improve healing at one year^{29,30}. However, the generalized treatment is often not suitable for certain patients and an individual evaluation must be done before giving a specific treatment, even giving a combination of different treatments if it is considered necessary.

Pressure ulcer (PU), also known as bedsore, decubitus ulcer or decubitus sore, is defined as "a surface of damage located on the skin and the underlying tissue caused by pressure, rubbing, friction or a combination thereof" (EPUAP 2003, European Pressure Ulcer Advisory Panel).

They may occur at any area of the body, being more common in bony protuberances (sacrum, hips and heels). PUs are relatively common in the hospital setting, especially affecting elderly patients suffering immobility, a serious acute pathology and/or neurological deficiencies.

The most important factor in the development of PUs is the pressure maintained. Parallel and/or tangential frictional forces, as well as a series of risk factors which fundamentally depend on the conditions of the patient of advanced age as discussed below, can be associated with this pressure.

PUs indicate the existence of microcirculation disorders in support areas of the body located on a hard surface. For that reason, the areas with bony protuberances are the most common areas for the onset of PUs. The pressure maintained on skin and soft tissues increases the interstitial pressure with lymphatic and blood vessel obstruction (microthrombi formation) leading to autolysis and the accumulation of toxic metabolic residues. The prolonged local ischemia leads to necrosis and the subsequent tissue ulceration both at the skin level and deep tissue levels. The hydrostatic pressure of the skin capillaries ranges between 16 and 32 mmHg. Any pressure exceeding these figures reduced the blood flow and can cause ischemic damage in even less than two hours.

The rubbing forces (frictional forces parallel to the epidermal surface) and the shearing forces (tangential forces which increase the friction in the pressure areas when the head of the bed is elevated more than 30°) reduce the pressure required to cause tissue damage.

A series of risk factors in the onset of PU have been described depending on the conditions of the patient of advanced age. Immobility is the most important factor of them all.

A review of epidemiological studies conducted in the United Kingdom, Canada and the U.S.A describes the reported prevalence of pressure ulcers in the United Kingdom between 4.4% and 37% depending on the type of institution. It is calculated that the prevalence in acute hospitals in Spain is 12%, leading to a high cost for the healthcare system, also generating an increase of work loads for nursing professionals.

Traditionally in Spain, the epidemiology related to pressure ulcers has been scarce and is generally local. However, in recent years, there has been a strong development in epidemiology awareness especially due to the support of scientific associations, the dimension that the problem has acquired and the awareness of healthcare administrators and professionals.

It is calculated that its incidence in the general population is 1.7% between 55 and 69 years of age and 3.3% between 70 and 75 years of age. There are no reliable data regarding its incidence in primary healthcare. In some series, it is estimated that 60% are developed in the hospital. More than 70% of PUs happen in people over 70 years of age.

Ulcers of this type are classified as:
- Grade 1 ulcers: the skin appears reddened, this reddening does not disappear upon removing the pressure that is exerted on the area.
- Grade 2 ulcer: the skin is chapped at the epidermis and dermis level causing the formation of blisters.
- Grade 3 ulcer: causes a loss of skin continuity with the onset of subcutaneous cell tissue necrosis forming a black scab clot called an eschar.
- Grade 4 ulcer: an extensive necrotic ulcer reaching the muscle, bone, and even vessels and nerves.

Finally, venous ulcers are wounds which generally happen on the legs and mostly occur in persons of advanced age. They are the main cause of chronic wounds, between approximately 70% and 90%³¹ of the cases. They usually occur in the internal supramalleolar region although they can also occur in the external area or be located slightly above the height of mid-calf. Their size is variable, and they are characterized by the lack of pain (except when they are infected). The skin surrounding the ulcer normally shows all the signs of venous insufficiency-induced skin disease. There are multiple theories as to their origin although it is possibly due to different causes. Currently, the most accepted theories as to their origin relate them with the triggering of a pre-ulcerous base of an ischemic process. So with the progression of venous hypertension, a high molecular weight protein exudation would be produced towards the outside of the vessels, accompanying red blood cell extravasation or following small local hemorrhages. These proteins will either be organized, as in the case of fibrinogen which is converted into fibrin, or they will be neutralized into other proteins, as occurs with growth factor-inhibiting alpha-2-macroglobulin. Venous hypertension would also cause leukocyte accumulation and local thrombosis phenomena of the venules. These physiopathological processes take place repeatedly with aging.

This will all result in the existence of an area around the vessels with a low growth factor content, the deficiency of which would cause the lack of tissue regeneration once the protective capacity of the epidermis is lost. Therefore, re-epithelialization and the formation of new dermis would be altered by the effect of such condition in persons of advanced age when the skin is broken.

Venous ulcers are more common in women with a man-woman ratio of 1-3. The typical incidence is after 65 years of age with a percentage of 5.6% of the population. They are therefore common in ambulatory or home nursing care. Once they are triggered, they tend to have an insidious progression with difficulty in healing, considerable involvement of the surrounding skin and frequent recurrences. Age is a fundamental risk factor, varicose veins occur four times more frequently and infection is seven times more frequent at 60 years of age.

The typical progression of venous ulcers is the following:
- Edema (the dilated veins generate valvular insufficiency whereby the venous return flow is slowed down and capillary hypertension with increased permeability thereof takes place, edema being generated), corona phlebectatica or telangiectasia (erythematous reaction in the ankle) occurs in the first stage.
- Sinuous and dilated veins, ochre or pigmentary dermatitis (due to the extravasation and deposition of hemosiderin), atrophie blanche (resulting from chronic capillaritis which in the long term causes the disappearance of blood capillaries, the appearance of whitish colored skin areas and dermal atrophy); cuff dermatosclerosis (due to chronic edema and fibroblast invasion, the tissues are transformed into a hard sclerotic band surrounding above and below the distal third part with edema, giving the appearance of "champagne bottle leg", lymphedema (elephantiasisis of the extremity), stasis eczemas (which cause itching), infections (exudates, cellulitis), hemorrhages, are detected in the second phase .
- The final phase corresponds to the onset of active ulcers.

In conclusion, the characteristics typical of venous ulcers are generally the following:
1. Pulses are present.
2. Variable size, from small to very extensive, often covering the entire leg.
3. They can be single or multiple (they tend to join together).
4. They are generally round- or oval-shaped although they can be irregular.
5. Their edges are smooth, somewhat raised, having a purplish-red color and are occasionally shiny, subsequently as they become chronic the edges become paler and harder.
6. The bottom of the ulcer depends on its stage and age, it is generally red due to congestion, although it can be yellowish if there is slough or necrosis. There may be purulent secretion signaling a secondary infection. When recovery is favored, it shows abundant granulation tissue.
7. In terms of pain, they are moderately painful, non-painful ulcers can be observed in clinic, but there are also other very painful ulcers that can generally be infected and accompanied by multiple periulcerative lesions such as those described.
8. Their common location is in the mid paramalleolar area, but their preferred location is in the internal lateral region of lower third part of the leg, i.e., the internal supramalleolar area.
9. Once they cover the entire leg, it is rare for them to affect the foot or thighs, but it is not uncommon for them to occur between the ankle and knee.
10. The circumstances surrounding venous ulcers delay the healing thereof, causing torpid wounds.
11. Postphlebitic ulcers with more excavated and irregular edges, often leading to the exposure of muscles and tendons, generally multiple in number, with significant skin disorders, have a worse prognosis due to the irreversible involvement of the irreplaceable deep system. In some cases, the lesion occurs several months after the thrombotic episode, although it most frequently occurs after about two years.
12. Furthermore, general factors such as anemia, diabetes, hypoproteinemia, obesity, unhealthy lifestyle and finally infections favor poor progression.

Unlike what happens in persons of advanced age, a normal healing or recovery of a wound requires suitable circulation, nutrition and immune condition, and negative mechanical forces must be avoided. Normally, it takes less than 14 days to complete the process and after hemostasis it has three phases that may overlap in time: hemostasis, inflammation, proliferation and remodeling with wound contraction.
a) Inflammatory phase: platelets aggregated and parenchymal cells damaged at the tissue lesion site secrete growth factors and other chemical mediators for healing wounds to attract and activate inflammatory cells and fibroblasts. Vasodilation and the increased permeability of the local capillaries allow transporting neutrophils to the wound site to phagocyte bacteria and wastes.
b) Proliferative phase: the re-epithelialization process starts within a few hours after wound formation. Epidermal cells detach from the basal membrane and migrate through the wound surface. The activated macrophages phagocyte wastes and release vascular endothelial growth factor (VEGF) and other growth factors to stimulate granulation tissue formation.
c) Remodeling: fibroblasts remodel the collagen matrix to enhance tissue strength and reduce wound thickness. During the second week of healing, the fibroblasts adopt a myofibroblast phenotype to facilitate the contraction of the scar which is being formed.

This wound healing and recovery process is evidently not reproduced in persons of advanced age. In the group of patients selected for the purpose of the present invention, a wound cannot properly heal in a suitable time interval. This is due to the result of the alteration of the time sequence and concentrations of biomolecules, such as proinflammatory cytokines, growth factors, proteases and protease inhibitors, which are key regulators of wound healing. Patients of advanced age therefore have a delayed healing and little response to conventional treatment.

### 1. Determination of the active ingredient and its concentration in the pharmaceutical formulations

As mentioned above, the only active ingredient (a.i.) of the pharmaceutical formulations used in the present invention is oleuropein with a degree of purity of at least 30%, preferably between 41.5% and 100%, specifically 41.5% and 80%.

Said active ingredient can be obtained with the desired degree of purity through conventional extraction and purification techniques from, among other sources, olive leaves and/or olive leaf extracts.

However, for the purpose of illustrating the present invention, the active ingredient oleuropein with a degree of purity of 41.5% was obtained from the commercial company Interquim, S.A. and the active ingredient with a degree of purity of 80% was obtained from the commercial company Extrasynthese, S.A (distributed in Spain through Cymit Química, S.L.).

The amount of active ingredient (oleuropein) to be used varies depending on two parameters: the desired concentration (in this case the concentrations produced were 10⁻¹ M, 10⁻² M, 10⁻⁴ M, 10⁻⁷ M, 10⁻⁹ M, 10⁻¹⁰ M and 10⁻¹¹ M) and the purity of the active ingredient oleuropein used (as mentioned, by way of example, oleuropein with two different purities was used in this study: 41.5% and 80%).

The concentrations are based on the molecular weight (MW) of oleuropein, which is 540.52 g/mol. Therefore, to prepare a solution of 10⁻²M, 5.4052 g/l, or in other words 0.54052g/100 ml, were weighed.

The active ingredient provided by Interquim, S.A. had a purity of 41.5%, therefore the amount of oleuropein necessary for preparing the pharmaceutical formulations according to the present invention is 13.02 g/l, or in other words 1.302g/100 ml.

The active ingredient provided by Extrasynthese had a degree of purity of 80%, therefore for the same oleuropein concentration in the formula (10⁻²M), 6.7566 g/l, or in other words 0.6757 g/100 ml, were necessary.

**Table 1. Amount of oleuropein necessary depending on the desired concentration and the purity of the active ingredient**

| Oleuropein: Molecular Weight 540.52 g / mol | | | |
|---|---|---|---|
| Concentration | Amount (in 100 ml) | Oleuropein purity (%) | Amount (in 100 ml) |
| 10⁻¹ | 5.4052 g | 41.5% | 13.025 |
| 10⁻¹ | 5.4052 g | 80% | 6.757 |
| 10⁻² | 0.5405 g | 41.5% | 1.3025 |
| 10⁻² | 0.5405 g | 80% | 0.6757 |
| 10⁻⁴ | 0.0054 g | 41.5% | 0.0013 |
| 10⁻⁴ | 0.0054 g | 80% | 0.0007 |

The remaining dilutions used (10⁻⁷ M, 10⁻⁹ M, 10⁻¹⁰ M, 10⁻¹¹ M) were obtained from these by means of serial dilutions.

### 2. Preparation of galenic gel, cream (o/w emulsion) and aqueous solution formulations

By way of example, the production method used for the concentration of 10⁻²M with an oleuropein purity of 41.5% is reproduced. The remaining concentrations were made following the same standard with the only variation in the amount of active ingredient incorporated which varied according to that described above (see Table 1) depending on the desired concentration and on the oleuropein purity of the base material.

### 2.1 Gel

The preparation of the gel consists of three phases:
First, the base of the gel is prepared; subsequently, the active ingredient is prepared; and finally both are mixed.

### 2.1.1. Ingredients

### 2.1.1.1. Ingredients for the preparation of 100 g of neutral carbomer gel

- Gelling agent: Carbopol 940: 1 g
- Antimicrobial preservative: Phenonip: 0.5% (between 0.25%-1%)
- Purified water for laboratory use qsf: 100 g
- Triethanolamine qsf pH 6.5
- Ethylenediaminotetraacetic acid or EDTA: 0.01%

### 2.1.1.2 Ingredients for the incorporation (dissolution) of the active ingredient

- Oleuropein: 1.3025 g (0.5405 g of 41.5% of purity)
- Propylene glycol: 5 g

### 2.1.2 Method for the preparation of the gel

### 2.1.2.1 Preparation of the gel base (neutral carbomer gel).

1. First, Phenonip and EDTA are dissolved in the purified water.
2. The previously disaggregated Carbopol 940 is then dispersed and the mixture is left to settle at 2-8°C (in a refrigerator) for 24 hours.
3. After 24 hours, the pH of the mixture is 3.5, so it does not have the appearance of a gel but rather a viscous liquid. Therefore, in order to start gelling and for the pH to be in an optimum range of 6-6.5, triethanolamine is added in a quantity sufficient (about 0.6%) for obtaining it and for completing the gelling.

### 2.1.2.2. Preparation of the active ingredient oleuropein

4. The oleuropein (0.5405 g) in powder form is dispersed in 5 g of propylene glycol using a mortar and a glass pistil. The incorporation of the active ingredient into the propylene glycol does not cause any problem and the result is a homogenous deep brown paste (the intensity varies depending on the degree of dilution).

### 2.1.2.3. Incorporation of the active ingredient into the gel base

5. The mixture of propylene glycol and active ingredient is then incorporated under vigorous stirring and at room temperature into the quantity of gel sufficient for obtaining 100 g.

A gel with the following proportions was thus obtained:

| Ingredients | Quantities (% by weight) |
|---|---|
| 41.5% oleuropein | 1.3025 (0.5405) |
| Carbopol | 1 |
| Phenonip | 0.5 |
| Triethanolamine | 0.6 |
| EDTA | 0.1 |
| Propylene glycol | 5 |
| Purified water for laboratory use | 91.4975 (qsf 100 ml) |

### 2.1.3. Justification of the ingredients used and alternatives thereof

### a) Gelling agent:

There are many gelling agents in addition to carbopol (carboxymethyl cellulose, hydroxypropyl methylcellulose, or even the already prepared bases (Hispagel®, LiquaGel® and others)). The first of them, Hispagel, was even used in the first two Phase I patients for the venous ulcer tests, in any event, the same proportion of a.i. oleuropein being added to the base as described in section 3.4.

Carbopol was chosen for its good stability over time, for its neutral character, because it presents perfect skin tolerance and for its stability against the vast majority of active ingredients. It would only be altered in the presence of ultraviolet light, but it does not present any preservation or oxidation problem when the package is closed.

Carbopol®: These are high molecular weight, anionic synthetic acrylic acid-based polymers. There are different types of Carbopol® that are designated by numbers, but the two most widely used in pharmacy today are: Carbopol® 934 and Carbopol® 940. Carbopol® 940 has been used in the present invention.

Description: virtually odorless, hygroscopic fine white powder forming small lumps. Solubility: it is slowly soluble (gels) in water, hardly soluble in ethanol and insoluble in chloroform and ether. aqueous disp. pH: 3.0-4.5 at 1%. Loss on drying: ≤ 2.0%. Viscosity of the gel at 0.5%: 40,000-60,000 cPs.

### b) Preservative:

Phenonip® (mixture of parabens in 2-phenoxyethanol) has been selected from the large amount of existing preservatives due to its broad spectrum against microorganisms and low toxicity. There are other very powerful preservatives which could also be used in the formula (e.g. 0.1% kathon), but Phenonip was preferred since the gel is meant for cutaneous use in an open wound.

Other preservatives suitable for the purpose of the present invention are mixtures of parabens (methylparaben and propylparaben), Quaternium-15 or the like.

### c) Solvent:

Propylene glycol was used because it improves the Carbopol® gel result by enhancing its spreadability. It in turn provides a better (more homogenous) incorporation of the active ingredient into the gel. The incorporation range is 1%-10%.

Another solvent suitable for the purpose of the present invention is glycerin.

### d) Alkalizing agent (pH regulator):

Triethanolamine has been used for the gel to be in the suitable pH range.

Other possible alkalizing agents for the purpose of the present invention are, for example, a 10% NaOH or a 10% KOH solution.

### e) Antioxidant:

Since the product is meant for application on open ulcers, no antioxidant has been added.

An antioxidant could optionally be used without affecting the action of the product, as was done according to the description provided in section 3.4 in the first two patients to whom oleuropein was applied in the study (patients 5 and 6). By way of example, the following should be mentioned: sodium bisulfite (E-223) (0.05%-0.1%) used in the aforementioned alternative formulations; ascorbic acid (vitamin C sodium E-301) (0.5%); vitamin E (0.5%); mixture of vitamins C and E (1:5 ratio) (0.5%), butylhydroxytoluene [E-321] (0.1%), etc.

If an antioxidant is included, it will be incorporated into the water before adding carbopol®.

### 2.2 Cream (o/w emulsion)

The preparation of the gel consists of three phases:
First, the base must be prepared; subsequently, the active ingredient must be prepared; and finally the a.i. is incorporated into the base.

### 2.2.1. Ingredients

### 2.2.1.1. Ingredients for the preparation of 100 g of aqueous emulsion base

- Neo PCL (a suitable range for cream would be 18% to 25% of Neo pcl depending on the desired consistency)
- Purified water for laboratory use in qsf 100 g (depending on the amount of NeoPcl, or antioxidant if it is incorporated, between 82% and 75%)
- Preservative: mixture of 0.2% nipagin and 0.2% nipasol (1:1 ratio)
- Antioxidant: 0.05% sodium bisulfite
- Trace metals sequestrant: EDTA (0.1%)

### 2.2.1.2 ingredients for the incorporation (dissolution) of the active ingredient

- 41.5% oleuropein: 1.3025 g (0.5405 g of oleuropein)
- Propylene glycol: 5 g

### 2.2.2 Method for the preparation of the o/w emulsion

### 2.2.2.1 Preparation of the base (aqueous emulsion)

1. First, 25% Neo Pcl (an optimal range would be between 18% and 25% depending on the desired consistency) is weighed.
2. The preservative (the mixture of 0.2% nipagin and 0.2% nipasol in a 1:1 ratio) is incorporated into the purified water for laboratory use, the proportion of which will be qsf 100 g (in this case 75%).
3. The purified water with the preservative and Neo Pcl are homogenized using a pistil or by means of mechanical stirring.

The three steps above would not be necessary if any of the self-emulsifying bases that are marketed were used; see the following sections "Ingredients used and alternatives thereof". The incorporation of the preservative in that case would be in point 4, homogenizing it together with oleuropein and sodium bisulfite in 5 g of propylene glycol.

### 2.2.2.2. Preparation of the active ingredient oleuropein

4. The a.i. oleuropein with a purity of 41.5% (solid in powder form) is dispersed together with sodium bisulfite (0.05%) in 5 g of propylene glycol using a mortar and a glass pistil for that purpose. The incorporation of the active ingredient into the propylene glycol does not cause any problem and the result is a homogenous deep brown paste (the intensity varies depending on the degree of dilution).

### 2.2.2.3. Incorporation of the active ingredient into the base

5. The mixture of propylene glycol and active ingredient is then incorporated under vigorous stirring and at room temperature into the quantity of base sufficient for obtaining 100 g.

An aqueous emulsion with the following proportions was thus obtained:

| Ingredients | Amount (% by total weight) |
|---|---|
| 41.5% oleuropein | 1.3025 (0.5405) |
| NeoPcl | 22.1475 |
| Preservative (nipagin and nipasol) | 0.4 |
| Purified water | 71 (qsf 100 ml) |
| Propylene glycol | 5 |
| Sodium bisulfite | 0.05 |
| EDTA | 0.1 |

### 2.2.3. Ingredients used and alternatives thereof

a) Bases:
   For aqueous emulsion-based samples, an emulsion prepared with Neo Pcl o/w was used.
   Neo Pcl® o/w incorporates ceteareth-10, beeswax, stearyl heptanoate, cetyl octanoate, spermaceti, myristoyl, dimethicone, mineral oil and 25% lanolin oil.
   Neo Pcl was used for its good properties and because it is excellent for incorporating active ingredients in addition to not causing allergic reactions. However, other self-emulsifying bases could have been used in addition to NeoPcl, such as, by way of example, the following: base F 2230 o/w; xalifin-15; or a polymeric emulsifier.
   Additionally, standard emulsions could have been used, such as, for example and in a non-excluding manner: Beeler base, lanette cream or cetomacrogol cream
b) Preservatives: In addition to the selected preservative, those described for the gel (including 0.5% phenonip) could have been chosen in a non-excluding manner.
c) Solvent: In addition to propylene glycol, those described for the gel could have been used in a non-excluding manner.
d) Antioxidants: They have not been included as it is a cream for application on an open ulcer. The antioxidants defined for the gel could optionally be included in a non-excluding manner.

### 2.3. Aqueous solution

### 2.3.1. Aqueous solution ingredients

- 1.301 g of active ingredient with a degree of purity of 41.5% (oleuropein)
- Preservative: 0.5% phenonip (or any of those used in the gel).
- Purified water qsf 100 g
- 5% propylene glycol (up to 10 g could be used depending on the desired thickness). It is not essential.

### 2.3.2. Method for the preparation of the aqueous solution for topical use

1. First, the preservative (phenonip) is mixed with the purified water for laboratory use (if an antioxidant such as metabisulfite is to be incorporated, it would be incorporated here).
2. The active ingredient is mixed with 5% propylene glycol with a pistil and is then added to water, and everything is stirred with a mechanical stirrer or rod.
3. Subsequently, the active ingredient is incorporated into the previous mixture of preservative and purified water by means of vigorous stirring with a mechanical stirrer or rod.
4. EDTA can optionally be added.

An aqueous solution with the following proportions was thus obtained:

| Ingredients | Amount (% by total weight) |
|---|---|
| 41.5% oleuropein | 1.3025 (0.5405) |
| Preservative (phenonip) | 0.5 |
| Purified water | 93.1975 (qsf 100 ml) |
| Propylene glycol | 5 |
| EDTA | 0.1 |

An antioxidant, by way of example, 0.05 g of sodium metabisulfite, could optionally be incorporated although any water-soluble antioxidant such as those mentioned for the gel would be valid.

Using the same standard method and ingredients, compositions at different active ingredient concentrations and degrees of purity of oleuropein (80%) were formulated according to Table 1. Said formulations were tested in the clinical tests which will be described below for the purpose of demonstrating the effectiveness of the compositions of the present invention in the treatment of specific diseases in the selected patient group.

### Examples: Clinical tests conducted in the selected patient group

The patients were selected after of a control visit conducted for the purpose of assuring compliance with the inclusion and exclusion criteria described below:

### 1. Inclusion criteria

The participating patients complied with each and every one of the following inclusion criteria:
1. Over 60 years of age.
2. Presence of inframalleolar, supramalleolar or leg (venous) ulcer or pressure ulcer that is chronic (at least 10 weeks of progression) and has not shown significant improvement (size reduction > 25%) in the 15 days prior to inclusion in the tests.
3. Granulated surface in the wound less than 70%³².
4. A/A index >0.7
5. Patients diagnosed with type 1 or 2 diabetes mellitus (DM) according to the American Diabetes Association (ADA) diabetes criteria.
6. No established diagnosis of infection in the ulcerated area based on clinical signs of either the ulcer, periulcerous lymphangitis, modified exudate characteristics, edema, or the systemic area, fever.
7. Patients with venous ulcer: FEDPALLA Grade equal to II-III.
8. Patients with diabetic foot: Grade I or II on the Wagner Scale.
9. Patients with PU: Stages 2-3 according to the PU Classification.

### 2. Exclusion Criteria

The patients did not comply with any of the following exclusion criteria:
1. Clinical signs or symptoms of local infection. Standard antiseptic treatments were applied in those patients presenting infection. The use of silver or cadexomer iodine dressings reduces the bacterial load and allows controlling the infection without significant local and/or systemic hypersensitivity reactions or lesion in healthy tissues. Both will be considered as the primary treatment intention³³.
   The use of silver sulfadiazine, mupirocin, fusidic acid and metronidazole are the only antibiotics recommended for topical use in ulcers and must be considered as the secondary intention and must always be used after using the antiseptic described above³³.
   Once the infection is eliminated, patients who complied with the remaining inclusion for the study were included in said study.
2. The presence of bone exposure at the bottom of the ulcer and/or touching bone by means of probe, and/or the presence of pathological fractures and/or bone sequestrations.
3. Patients with HbA1C values > 9% or A/A index < 0.7 (where appropriate).
4. Presence of co-morbidities representing an increase of risk according in the investigator's opinion.
5. Patients with a serious disease of any etiology.
6. Patients with hepatic insufficiency (with AST and/or ALT values > 5 times the normal value established in the reference ranges of the hospital's local laboratory).
7. Patients with serious renal insufficiency (creatinine clearance <30 ml/min).
8. Patients with connective tissue diseases.
9. Alcoholics and smokers (to avoid confounding factors).
10. Certain medications can also be confounding, including some antibiotics (clarithromycin, doxycycline, tetracycline), non-steroidal anti-inflammatory drugs (aspirin, ibuprofen), COX-2 inhibitors (celecoxib), diuretics (bumetanide, furosemide), antihypertensives (captopril, enalapril, metoprolol), systemic corticosteroids, immunosuppressants, antiarthritics (etanercept, infliximab), antidiabetics (pparγ agonists: pioglitazone, rosiglitazone), pentoxifylline, becaplermin (Regranex). Patients following these treatments were excluded, possibly being included after a drug wash-out phase of at least 30 days before their inclusion in the study.
11. Pregnant women or women of child bearing age who do not use an effective contraceptive method, women who are breastfeeding.
12. Known hypersensitivity to any of the components included in the treatments used in the study.
13. Given the great influence of nutrition on the ulcer recovery process³⁴, those who were unable to maintain a suitable balanced diet were excluded.

### 3. Description of the treatment

The patients were treated according to the type of ulcer, depending on whether they were venous ulcers, diabetic foot ulcers or pressure ulcers.

### 3.1. Recovery-related procedures

The design of the study was pragmatic, the recovery-related procedures during the study period were the same as those received by the patients before starting the clinical test, both for the so-called control patients and for the patients treated with oleuropein.

The difference between both groups was the application of the products containing oleuropein in the patients under the test treatment once the wound recovery-related procedure is performed and before bandaging the area in the case of venous ulcers.

### Frequency of the recovery-related procedures:

The recovery-related procedures were performed every 12, 24 ,48 hours according to the design of the study in each case. Preferably the recovery-related procedure was used every 24 hours. The recovery-related procedures were performed either by the nurse or by the patient's family members following the nurse's instructions.

### Debridement:

Whenever it was deemed necessary, before applying the wound recovery-related procedure, a debridement of the area was performed.

### Wound cleansing:

The wound cleansing was identical to the one the patients had been following under medical treatment before starting the clinical test:
The edges of the wound were cleansed using gauze soaked in warm water and soap or physiological saline. The area was subsequently rinsed with abundant physiological saline. The wound was cleansed using to that end gauzes soaked in physiological saline. Washing by entrainment were performed in those cases in which fibrin was present, and the area was rinsed with abundant physiological saline. Finally, the edges were carefully dried with sterile gauzes.

### 3.2. Specific treatments

All the patients had been treated with polyurethane foams, calcium alginate dressings or hydrogels. Before starting the treatment and for at least 15-20 days, the processes were standardized, the ulcer having been occluded in all of them with silicone dressings. Additionally, in those patients with ulcers of a venous etiology, compressive treatment was always applied.

Three basic therapeutic groups were established:
a) Control: the treatment described above was continued (recovery-related procedures and occlusion of the ulcer with silicone dressings).
b) Control plus hyaluronic acid: In patients with venous ulcers. The treatment described above was continued (control group), adding 0.2% hyaluronic acid (sodium salt) [Jaloplast®], applying a thin layer daily before placing the silicone dressing.
c) Control plus becaplermin (platelet derived human recombinant growth factor BB): In patients with neuropathic ulcers or pressure ulcers. The treatment described above was continued (control group), adding 0.01% becaplermin [Regranex Gel®], applying a thin layer daily before placing the silicone dressing.
d) Treatment with oleuropein: The treatment described above was continued (control group), adding oleuropein at different dosages and time schedules appropriately specified below, applying a thin layer before placing the silicone dressing.

No modification was introduced in the described treatment regimens.

### 3.3. Duration of the study. Assessment time chart

Follow-up period: 6 weeks or until complete healing of the ulcer.
Number of measurements: Six measurements distributed in the following manner were carried out:
- Measurement 0: Day -15 (recruitment), test for confirming compliance with inclusion conditions
- Measurement 1: Start of the trial (day 0)
- Measurement 2: Day 14 (+/- 2 days)
- Measurement 3: Day 28 (+/- 2 days)
- Measurement 4: Day 35 (+/- 2 days)
- Measurement 5: Day 42 (+/- 2 days)

### 3.4. Experimental drug used

Pharmaceutical formulation as described above comprising oleuropein as the only active ingredient with a percentage of purity of the a.i. of 41.5% or 80%; applied through the gel, o/w emulsion/cream or aqueous solution carriers, prepared for use at concentrations and in application regimens as indicated in the present invention.

Variants of the clinically tested formulations:
As mentioned above, the a.i. was applied on the carrier gel in the first patients included in Phase I of the venous ulcer study (patients 5 and 6), using to that end instead of the preparation shown in section 2.1, an already commercially prepared gel base (Hispagel), diluted to 50% with water to make the application easier. For the remaining patients to whom gel was applied in the treatment, the ingredients and the production standard described in section 2.1 were used.

A gel with the following proportions was thus obtained:

| Ingredients | Amounts (% by weight) |
|---|---|
| 41.5% oleuropein | 1.3025 (0.5405) |
| Phenonip | 0.5 |
| Sodium bisulfite | 0.05 |
| Propylene glycol | 5 |
| Hispagel (diluted to 50%) | 46.57 |
| Purified water for laboratory use | 46.57 (qsf 100 ml) |

### 4. Methods for evaluating product response

### 4.1 Method for measuring the ulcer area and the remaining parameters:

For each patient, the profile of the ulcer was drawn on sterilized transparent paper for each of the measurements. To that end, STAEDTLER permanent DRY SAFE "F" markers were used.

The ulcer surfaces were calculated by means of standard planimetry, importing the outlines obtained to the AUTOCAD 2011 program whereby the surface and length of the outer perimeter of each ulcer in each of the measurement milestones was calculated. The remaining evaluated parameters (moisturizing, dermatitis, vascularization, edges, deposits in the ulcer) could be directly seen by the naked eye and their progression was noted in each measurement milestone.

### 4.2. Main variables for evaluating treatment efficacy

### 4.2.1. Wound recovery rate

The wound recovery rate was evaluated by means of the following equations^{35,36}:

### Scale 1

### Percentage variation of the ulcer surface

Av = (A0-A1)/A0, wherein Av is the variation in the ulcer area, A1 the area in the reference control, A0 the area in the previous control (start of the study).

It expresses the percentage reduction of the ulcerated area.

### Scale 2

### Percentage variation of the ulcer perimeter:

Pv = (P0-P1)/P0, wherein Pv is the variation the ulcer perimeter, P1 the perimeter in the reference control, P0 the perimeter in the previous control (start of the study).

It expresses the percentage reduction of the ulcer perimeter.

### Scale 3

### Equation as a function of the area (daily reduction of the ulcerated area)

Av = (A0-A1)/t, wherein Av is the variation in the ulcer area, A1 the area in the reference control, A0 the area in the previous control (start of the study) and t the time variable between A0 and A1 expressed in days.

It expresses the daily reduction of the ulcerated area.

No calculations were performed for the progression of the ulcer volume because despite allowing the assessment of the progression of the granulation tissue in the ulcer, the variability in the interpretation of the results is high and performing the study of the ulcer volume systematically is not recommended^{36,37}.

### 4.2.2. Generally accepted ulcer diagnosis assessment scales (FEDPALLA, Wagner and PU Studies).

### 4.2.2.1 Scale 4.1. Perilesional skin assessment: FEDPALLA assessment scale

The FEDPALLA assessment scale³⁸ is used to assess perilesional skin. The scale contemplates 5 variables and there are 5 parameters within each variable (Table 2) with a score of 5 to 1. The sum of each parameter gives a score referring to the prognosis grade for epithelization (Table 2).

**Table 2**

| Moisturizing | Dermatitis | Vascularization | Edges | Deposits | Score |
|---|---|---|---|---|---|
| Normal skin | Normal skin | Red erythema | Smooth | Squamae | 1 |
| Macerated 1 cm | Dry eczema | Purplish erythema | Inflamed and mamelons | Crusts | 2 |
| Macerated >1 cm | Exudative eczema | Black-bluishbrown | Rounds or excavated | Hyperkeratosis | 3 |
| Dry | Vesicular eczema | Erythema >2 cm and heat (cellulitis) | Sclerosed | Seropurulent pustules | 4 |
| Dry + sclerosis | Eczema with erosion or lichenified | Black (thrombosed) | Necrosed | Edema, lymphedem a | 5 |

The grades go from lower to higher in relation to the score obtained with the sum of the parameters to be taken into account for the perilesional skin and which are inversely proportional thereto. In other words, the higher the score, the lower the grade and the better the prognosis for epithelization (Table 3).

**Table 3**

| Points | Grades | Epithelization prognosis |
|---|---|---|
| 21-25 | IV | Very bad |
| 16-20 | III | Bad |
| 11-15 | II | Good |
| 5-10 | I | Very good |

### 4.2.2.2 Scale 4.2. Clinical evaluation of diabetic foot

In the clinical evaluation of diabetic foot, the Wagner-Merrit³⁹ classification (Table 4) which allows categorizing the lesions depending on the depth of the ulcer and has shown a high correlation with the tendency for healing is normally used.

**Table 4**

| Grade | Lesion | Characteristics |
|---|---|---|
| 0 | None, foot risk | Thick calluses, prominent metatarsal heads, claw toes, bone deformities. |
| 1 | Superficial ulcers | Complete destruction of skin thickness |
| 2 | Deep ulcers | Penetrate the skin, fat, ligaments without affecting bone, infected |
| 3 | Deep ulcers plus abscess | Extensive, deep, secretion and bad odor |
| 4 | Localized gangrene | Necrosis of part of the foot |
| 5 | Extensive gangrene | Entire foot affected, systemic effects |

### 4.2.2.3. Scale 4.3 Pressure Ulcers (PU) classification

This classification was used because it is the most accepted classification on national and international levels both by the *Grupo Nacional para el Estudio y Asesoramiento sobre las Úlceras por Presión* (GNEAUPP) (National Pressure Ulcer Study and Advisory Group) and by the European Pressure Ulcer Advisory Panel (EPUAP).⁴⁰ It is the classification defined by the North American Agency for Healthcare Research and Quality (AHQR) in 1989 and is based on the classification of grades (I-IV) created by Shea in 1975 and modified to stages (0-IV) by Torrance in 1983, removing the pre-ulcer stage:

### Stage I

Pressure-related observable alteration on the entire skin which manifests itself by a skin erythema which does not turn pale upon pressing, in dark skin, it may have red, blue or purple hues. In comparison with an area adjacent to or opposite the body which is not subjected to pressure, it may include changes in one or more of the following aspects: skin temperature (hot or cold), tissue consistency (edema, induration) and/or feelings (pain, burning).

### STAGE II

Partial loss of skin thickness affecting the epidermis, dermis or both. Superficial ulcer having the appearance of an abrasion, blister or superficial crater.

### STAGE III

Complete loss of skin thickness involving a subcutaneous tissue lesion or necrosis which may extend downwards but not through the underlying fascia.

### STAGE IV

Complete loss of skin thickness with extensive destruction, tissue necrosis or muscle, bone or support structure lesion. It may have cavities, tunneling or winding trajectories.

### 4.3. Secondary variables

### 4.3.1. Scale 5. Ease of application

The nurses responsible for the treatment must compare the ease of application of each of the products (galenic gel, cream or solution formulations) with respect to other similar products. To that end, they must grade the product on a scale of 1 to 10 as described below:
- Very convenient with respect to other similar products (9 or 10 out of 10)
- Convenient with respect to other similar products (7 or 8 out of 10)
- Indifferent with respect to other similar products (5 or 6 out of 10)
- Inconvenient with respect to other similar products (3 or 4 out of 10).
- Very inconvenient with respect to other similar products (1 or 2 out of 10).

### 4.3.2. Scale 6. Safety

A plan was drawn up for a record of potential adverse or toxicity reactions during the study. No significant data were collected in this sense.

### 5. Assessment of the efficacy, safety and applicability of the product (objectives)

### Success variables (product efficacy)

### Objectives

a) According to scale 1. "Accumulated variation of the ulcer surface in relative terms".
   1. Objective 1: Complete wound recovery (100% diameter reduction) or greater than 70% within the study period (week 6).
   2. Objective 2: Healing of the surface exceeding controls by 30 percentage points (20 points if the patient has one grade more in the FEDPALLA, Wagner or PU scales).
b) According to scale 2. "Accumulated variation of the ulcer perimeter in relative terms".
   3. Objective 3: Complete healing of the ulcer perimeter (100% reduction) or greater than 70% within the study period (week 6).
   4. Objective 4: Perimeter reduction exceeding controls by 30 percentage points (20 points if the patient has one grade more in the FEDPALLA, Wagner or PU scales).
c) According to scale 3. "Equation as a function of the area (expressed as the percentage of daily reduction of the ulcerated area)".
   5. Objective 5: Percentage of daily reduction of the ulcer area in patients treated with oleuropein is 30% greater than the percentage of daily reduction in control patients.
d) According to scale 4 (4.1, 4.2, 4.3). "Reduction of a grade or stage in the appropriate generally accepted assessment scale for ulcers diagnosis (FEDPALLA -venous ulcer-, Wagner -neuropathic ulcer- and PU stages -PU-)"
   6. Objective 6: To achieve the reduction from grade III to grade I ulcers or complete recovery. To achieve the reduction from grade II ulcers to complete recovery.
e) According to scale 5. "Ease of application"
   7. Objective 7: To achieve a mean score equal to or greater than 7 points (convenient or very convenient application).
f) According to scale 6. "Safety"
   8. Objective 8: Absence of adverse or toxicity reactions.

### 6. Development and clinical tests

Clinical tests in humans conducted on a total of 48 patients were performed by following the order below:
1. Venous ulcers
2. Diabetic foot
3. Pressure ulcers (PU)

The tests conducted for each therapeutic application are described below:

### 6.1 Venous ulcers

Clinical studies conducted in patients with venous ulcers were exhaustively performed on 36 patients, divided into six different phases with different methods and objectives:

### Phase I. Evaluation of the level of efficacy, applicability and safety of oleuropein produced at a concentration of 10⁻² under the galenic gel, cream (o/w emulsion), aqueous solution forms.

### Specific objectives:

To check the efficacy of the different galenic forms against the control as well as to determine if any of them show an efficacy that is lower than the rest.

### Execution:

Several batches were produced on the gel, cream and solution carriers with an identical active ingredient concentration (10⁻²M). This concentration was chosen to start the clinical tests as it is the highest concentration previously tested in rats. In other words, a criterion of caution was adopted.

Ten patients were selected for this first phase of the clinical study. All of them complied with the inclusion criteria in the study described above:
- Patients 1 and 2: Control. Application of the standard recovery treatment according to section 3.1 above.
- Patients 3 and 4(*): Control + Hyaluronic acid. Application of the treatment with hyaluronic acid described in section 3.2 above.
- Patients 5 and 6: Application of a gel at a concentration of 10⁻² M every 24 hours
- Patients 7 and 8: Application of a cream at a concentration of 10⁻² M every 24 hours
- Patients 9 and 10: Application of a solution at a concentration of 10⁻² M every 24 hours

As can be observed, the product was applied on all patients every 24 hours to enable establishing a comparison.

(*) These two patients were included in order to demonstrate that the claimed compositions with oleuropein are better than the current state of the art in terms of efficacy.

### Conclusions:

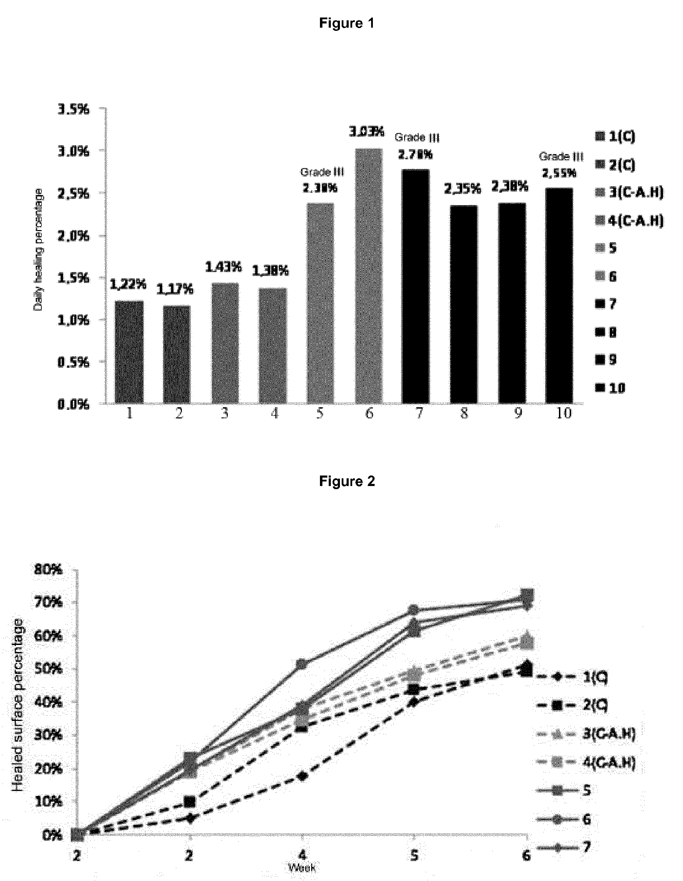

1. The use of oleuropein through topical route in any of the three galenic forms used showed a high degree of efficacy (it offered much better results than those of the two control groups which represent the current state of the art, and healed chronic ulcers without a significant improvement in the preceding two weeks).
2. All the main objectives (referring to the reduction of the area, of the perimeter, to the daily healing rate and the reduction by one or more grades of the FEDPALLA scale) were met by the six patients to whom oleuropein was administered regardless of the galenic formula used. It should be pointed out that the average daily healing rate in patients to whom the product object of the present invention was administered was 98.71% higher than (almost double) the average healing rate of the four control patients, including the two patients treated with hyaluronic acid. See Figure 1.
3. Regardless of the foregoing, the results obtained by the product in gel form showed a slightly higher recovery speed as well as a greater ease of application according to the nurses applying them, who assessed the use of the gel as "very convenient" (9.5 out of 10), and the use of the cream and the aqueous solution (7.5 and 7 out of 10, respectively) as "convenient". None of the three galenic forms used caused discomfort or toxicity problems in the patients.

### Phase II. Evaluation of the level of efficacy of the oleuropein produced under the galenic gel, cream (o/w emulsion), aqueous solution forms with minimum oleuropein concentration with efficacy previously tested with success:

### 10⁻¹⁰ M

### Specific objectives:

The objective of this second test was to demonstrate the efficacy and superiority of the treatment with all the galenic forms previously tested with success (gel, cream and solution) over the state of the art by applying the minimum active ingredient concentration already used previously with success in cells (10⁻¹⁰ M) in clinical tests. Additionally, an objective was to compare the efficacy of this very diluted concentration with respect to the concentration of 10⁻² M as well as to compare the efficacy of the different galenic formulations with one another.

### Execution:

Products were produced for this purpose in conditions similar to those described in Phase I with the aforementioned variation in their oleuropein concentrations (10⁻¹⁰ M) (see section 3.4) since the lowest active ingredient concentration levels tested in previous steps of the research were used.

Three new patients were selected for the study (patients 5, 6 and 7) because the same four patients described in Phase I were used as controls. All of them complied with the inclusion conditions described above:
- Patients 1 and 2: Control. Application of the standard recovery treatment according to section 3.1.
- Patients 3 and 4: Control + Hyaluronic acid. Application of the treatment with hyaluronic acid described in section 3.2.
- Patient 5: Application of a gel at a concentration of 10⁻¹⁰ M every 24 hours
- Patient 6: Application of a cream at a concentration of 10⁻¹⁰ M every 24 hours
- Patient 7: Application of a solution at a concentration of 10⁻¹⁰ M every 24 hours

As can be observed, the product was applied to all the patients every 24 hours to enable establishing a comparison.

### Conclusions:

The different formulations with an oleuropein concentration of 10⁻¹⁰ M showed a level of efficacy very similar to each other, especially after treatment week 5 (see Figure 2), repeating the pattern already observed in Phase I where the healing levels of the gel are slightly greater (1.72% daily healing versus 1.68% and 1.65% of the cream and solution) probably due to the greater ease offered by this medium for the absorption of the active ingredient.

The three formulations (gel, cream, solution) showed results that were worse than those shown for the more concentrated formulations:
- 2.58% mean daily reduction in Phase I (concentration of 10⁻² M) versus the average 1.68% in Phase II (concentration of 10⁻¹⁰ M).
- 99.27% mean healing at the end of the study (week 6) in Phase I versus 70.70% in Phase II.

In any case, the efficacy of the products with this active ingredient concentration and purity was much higher than the controls. Again, no patient showed adverse effects or anomalies that may have been related to the applied product.

It is therefore very reasonable to think that under identical production conditions, the galenic formulations in gel form, cream form, solution form or the like which include the active ingredient of the present invention with a degree of purity of at least 30%, preferably between 41.5% and 100%, specifically 41.5% and 80% in concentrations comprised between the two extremes the efficacy of which has been tested with success (10⁻² M and 10⁻¹⁰ M), will have levels of efficacy similar to those effectively tested.

In relation to the foregoing and for the purpose of checking if the product was also effective at higher concentrations, Phase III of the clinical tests, including tests with the three galenic formulations concentrated at 10⁻¹ M, was carried out.

### Phase III. Evaluation of the level of efficacy of the oleuropein produced under the galenic gel, cream (o/w emulsion), aqueous solution forms with maximum oleuropein concentration (10⁻¹ M)

### Specific objectives:

To check the efficacy of the treatment with all the galenic forms previously tested with success (gel, cream and ointment) by applying a high active ingredient concentration (10⁻¹ M), as well as to check its efficacy with respect to the concentrations already tested in Phases I and II.

### Execution:

Products were produced for this purpose in conditions similar to those described in Phases I and II with the aforementioned variation in their oleuropein concentrations (10⁻¹ M). See sections 2 and 3.4.

In addition to the four aforementioned control patients, three new patients, all of whom complied with the inclusion conditions described above in the present invention, were selected:
- Patients 1 and 2: Control. Application of the standard recovery treatment according to section 3.1.
- Patients 3 and 4: Control + Hyaluronic acid. Application of the treatment with hyaluronic acid described in section 3.2.
- Patient 5: Application of a gel at a concentration of 10⁻¹ M every 24 hours
- Patient 6: Application of a cream at a concentration of 10⁻¹ M every 24 hours
- Patient 7: Application of a solution at a concentration of 10⁻¹ M every 24 hours

As can be observed, the product was applied to all patients every 24 hours to enable establishing a comparison.

### Conclusions:

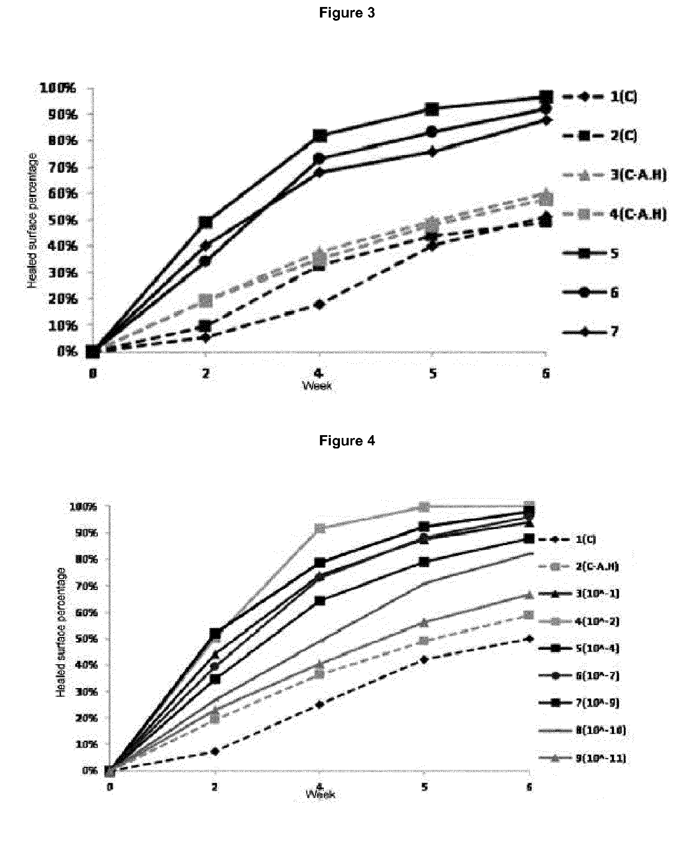

The different formulations with an oleuropein concentration of 10⁻¹ M with a degree of purity of at least 30%, preferably between 41.5% and 100%, specifically 41.5% and 80%, have a level of efficacy very similar to one another and much greater than the controls in all cases, the gel formulation of the product being the formulation that again minimally presented again the highest level of efficacy. See Figure 3.

Therefore, once Phases I, II and III ended, it is very reasonable to think that under similar production conditions, the galenic formulations in gel form, cream form, aqueous solution form or the like which include oleuropein at concentrations comprised between the two extremes the efficacy of which has been tested with success, 10⁻¹ M and 10⁻¹⁰ M (although the lower extreme of concentrations was replaced by 10⁻¹¹ M after being tested with success subsequently in Phase IV), will have levels of efficacy similar to those effectively tested in patients.

In any case, the next phase (Phase IV) of the clinical study further examined this matter since it consisted of testing, for the gel form, a broader spectrum of applicable active ingredient concentrations comprised within the range. The tests were not performed in a larger number of galenic forms, concentrations and patients solely due to the difficulty in recruiting a sufficient number of patients who complied with the study inclusion conditions, especially considering that Phases V and VI of the clinical tests on venous ulcers for which a significant number of patients would be needed were still pending.

In terms of the secondary objectives of the study, the nurses repeated the same observations: the application of the gel was shown to be slightly more convenient, although the cream and the solution did not present any problem for use. Regarding safety, like in the remainder of the study, anomalies were not presented in terms of adverse or toxicity reactions.

### Phase IV. Evaluation of the level of efficacy of the galenic gel formulation in virtually all the concentrations of the range in which the active ingredient is effective: 10⁻¹, 10⁻²,10⁻⁴,10⁻⁷,10⁻⁹,10⁻¹⁰,10⁻¹¹.

Once the efficacy of the product in its gel, cream and solution forms in two extremes of the concentration range in which oleuropein has a healing effect has been demonstrated, and having taken into account the difficulty for recruiting patients who complied with the strict requirements established for forming part of the study, Phase IV of the clinical tests only included tests with the galenic gel form.

### Specific objectives:

To demonstrate the efficacy of the active ingredient oleuropein with a degree of purity of at least 30%, preferably between 41.5% and 100%, specifically 41.5% and 80% at different concentrations, and especially at the concentration of 10⁻¹¹ M, which is outside the previously tested range. To that end, this Phase IV of the clinical test was carried out only, and by way of example, in the gel medium.

The results obtained with the gel carrier can be extrapolated to the remaining galenic formulations taking into account the high similarity existing between the results of the different tested formulations (gel, cream, solution) for the three previously tested concentrations (10⁻¹ M, 10⁻² M and 10⁻¹⁰ M). Therefore, the obtained results must not be understood as being attributable solely to the formulation in the gel medium, but rather this formulation has been selected only by way of example.

### Execution:

The production of the compounds was similar to that shown in the previous phases with the obvious exception of the active ingredient concentration used (see sections 2 and 3.4).

The objective was to provide the data of two patients for each of the different concentrations to be tested. The tested concentrations were 10⁻¹ M, 10⁻² M, 10⁻⁴ M, 10⁻⁷ M, 10⁻⁹ M, 10⁻¹⁰ M and 10⁻¹¹ M (10⁻² M has already been tested previously in Phase II, but the results were included again for comparison purposes). Therefore, and taking into account that the concentrations of 10⁻¹ M and 10⁻¹⁰ M have already been tested in respective patients during Phases III and II, respectively, and that the concentration of 10⁻² M has already been tested in two patients in Phase I, the distribution of patients for this phase was as follows:
- Patients 1 and 2: Control. Application of the standard recovery treatment according to section 3.1.
- Patients 3 and 4: Control + Hyaluronic acid. Application of the treatment with hyaluronic acid described in section 3.2.
- Patients 5 and 6: Application of a gel at a concentration of 10⁻¹ every 24 hours (patient 5 was previously included in Phase III).
- Patients 7 and 8: Application of a gel at a concentration of 10⁻² every 24 hours (both patients were previously included in Phase I).
- Patients 9 and 10: Application of a gel at a concentration of 10⁻⁴ M every 24 hours
- Patients 11 and 12: Application of a gel at a concentration of 10⁻⁷ M every 24 hours
- Patients 13 and 14: Application of a gel at a concentration of 10⁻⁹ M every 24 hours
- Patients 15 and 16: Application of a gel at a concentration of 10⁻¹⁰ M every 24 hours (patient 15 was previously included in Phase II).
- Patients 17 and 18: Application of a gel at a concentration of 10⁻¹¹ every 24 hours

As can be observed, the product was applied to all patients every 24 hours to enable establishing a comparison.

### Conclusions:

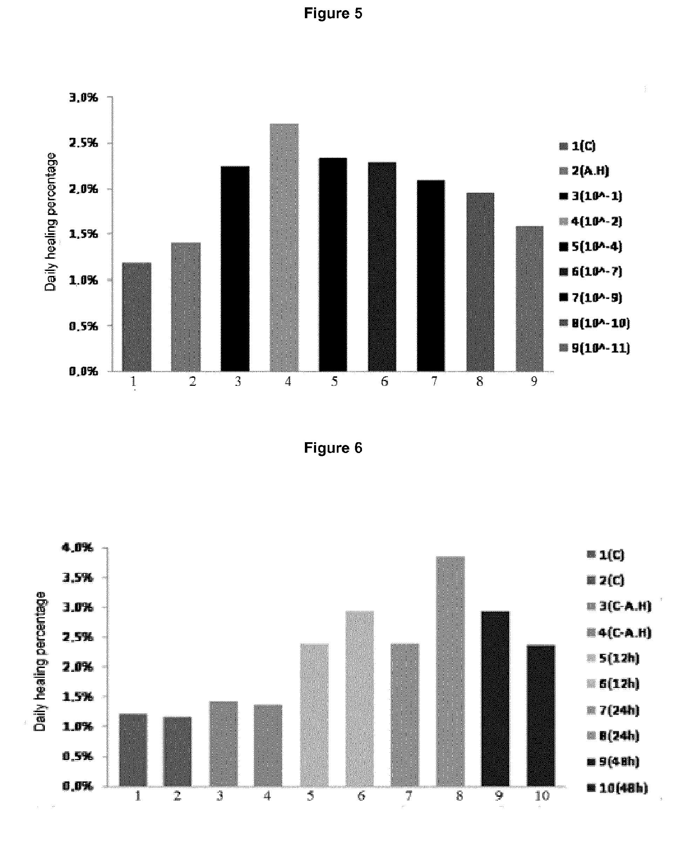

Phase IV of the study demonstrated that oleuropein is effective and better than the controls under any of the measurement scales established in section 4 in the entire product concentration range comprised between 10⁻¹ M and 10⁻¹¹ M. See Figure 4. It was also seen that the maximum efficacy of the active ingredient is achieved under the concentration of 10⁻² M (even above 10⁻¹ M) and very progressively decreases until 10-¹¹ M, the efficacy of which is only slightly better than the controls. See Figure 5.

As mentioned in the previous phases, and having taken into account the correlation existing between the results obtained for the gel, cream and solution formulas for the concentrations of 10⁻¹ M, 10⁻² M and 10⁻¹⁰ M, it is logical to think that the results obtained in the new concentrations tested only in gel form can be extrapolated to the remaining formulations. In terms of the secondary variables of the product assessment, the a.i. oleuropein was once again, and this time with a higher patient population, i.e., ten new patients, confirmed as not having adverse effects, in addition to the fact that the use thereof in gel form is easy to apply by the nurses responsible for the treatments (mean score of 8.33 out of 10).

### Phase V. Evaluation of the level of efficacy of the product (in its gel form) under different application regimens (every 24 hours vs. every 12 hours vs. every 48 hours). Objective:

To check the efficacy of the active ingredient oleuropein under different application regimens since the product had only been applied every 24 hours up until that time.

### Execution:

To that end, Phase V of the clinical test was carried out, by way of example, in the gel medium as more data are available about it and it was the most effective up until that time. The preparation of the product was similar to that for Phase II (see sections 2 and 3.4)

Four new patients who complied with the inclusion requirements described above were chosen for this part of the study. To that end and taking into account that the concentration of 10⁻² M applied every 24 hours had already been tested during Phase II, the distribution of patients for this phase of the study was as follows:
- Patients 1 and 2: Control. Application of the standard recovery treatment according to section 3.1.
- Patients 3 and 4: Control + Hyaluronic acid. Application of the treatment with hyaluronic acid described in section 3.2.
- Patients 5 and 6: Application of a dose of gel at a concentration of 10⁻² M every 12 hours.
- Patients 7 and 8 (from Phase I): Application of a dose of gel at a concentration of 10⁻² M every 24 hours.
- Patients 9 and 10: Application of a dose of gel at a concentration of 10⁻² M every 48 hours.

The recovery-related procedures were always performed before each application of the product (every 12, 24 or 48 hours).

### Conclusions:

This phase of the study served to test if oleuropein with a degree of purity of at least 30%, preferably between 41.5% and 100%, specifically 41.5% and 80%, is better than the controls under any application regimen, even with applications every 48 hours. See Figure 6. Additionally, it was verified that there were no significant differences in the efficacy of the a.i. among the different application regimens tested (12 hours, 24 hours, 48 hours).

All the objectives indicated for evaluating the efficacy, ease of application and safety of the product were met under the three application regimens. It should be mentioned that none of the patients to whom the product was applied every 12 hours, with the consequent increase of direct handling of the ulcer, showed any sign of infection or adverse reaction. This pattern was repeated in patients with neuropathic ulcers or pressure ulcers to whom the a.i. was applied every 12 hours.

### Phase VI. Evaluation of the level of efficacy of galenic formulations starting from two active ingredients with different degrees of purity Specific objectives:

To check that there are no significant variations in effectiveness results of the pharmaceutical compositions in the purity range of the active ingredient of the present invention used in the formulation of the pharmaceutical compositions.

### Execution:

To perform this performance comparison, a batch of gels, all of them with a concentration of 10⁻⁴ M, was produced (the batch was produced again, by way of example, in the gel medium as more data are available about it and it was the most effective up until that time). The preparation was carried out by following the standards described taking into account the different a.i. concentration in the extract (see sections 2 and 3.4).

Six new patients who complied with the requirements described above for their inclusion were chosen for this part of the study. The results obtained for these patients were compared with those obtained by the two patients treated in Phase IV with the gel at 10⁻⁴ M which included the a.i. with a degree of purity of 41.5%, as well as with the four control patients.

The patients included in this phase of the study are described below:
- Patients 1 and 2: Control. Application of the standard recovery treatment according to section 3.1.
- Patients 3 and 4: Control + Hyaluronic acid. Application of the treatment with hyaluronic acid described in section 3.2.
- Patients 5 and 6 (from Phase IV): Application of a gel with an a.i. concentration of 10⁻⁴ M containing oleuropein with a degree of purity of 41.5% every 24 hours.
- Patients 7, 8, 9, 10, 11 and 12: Application of a gel with an a.i. concentration of 10⁻⁴ M, containing oleuropein with a degree of purity of 80% every 24 hours.

### Conclusions:

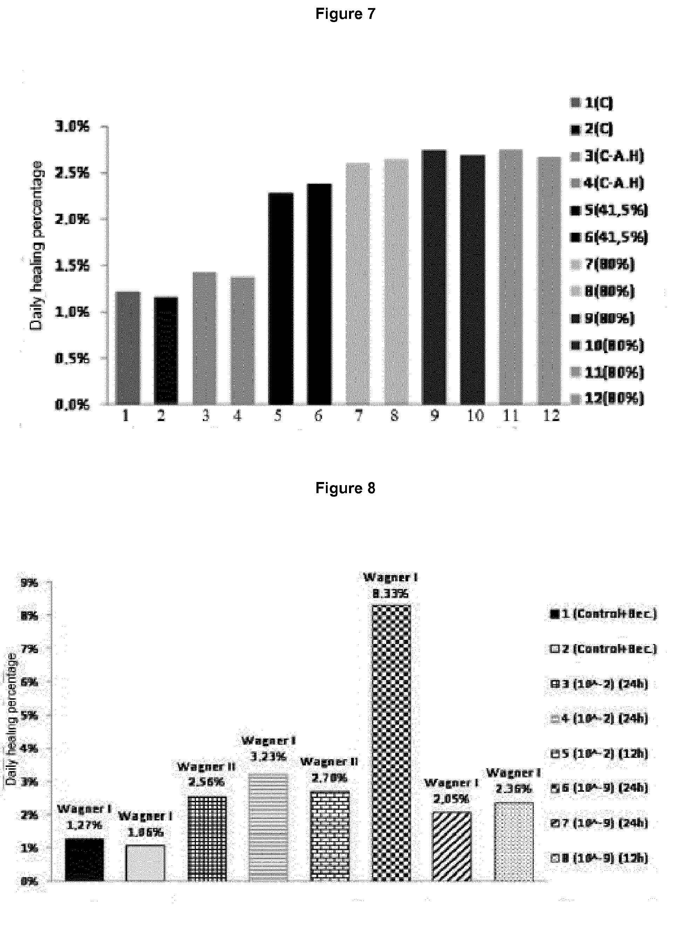

It has been demonstrated that there are no very significant differences among the results obtained with the gels incorporating the a.i. with a degree of purity of 41.5% and those incorporating the a.i. with a degree of purity of 80%, although the latter showed a faster curing speed (average daily healing of 2.33% for ulcers treated with 41.5% oleuropein versus 2.69% of 80% oleuropein). The healing effect of the purest a.i. is furthermore much better than the controls (see Figure 7). Therefore, it can be concluded that the healing effect of the product is positive and it increases as the purity of the active ingredient used in the formulation of the pharmaceutical compositions of the present invention increases.

All the objectives of the evaluation of efficacy were met by this product which incorporated a purer a.i. Similarly, irritations or infections did not occur in patients treated with pure oleuropein, which confirms the safety of this a.i. with the claimed degree of purity.

### 6.2. Neuropathic ulcers (diabetic foot)

Once the efficacy of the product of the present invention has been demonstrated on venous ulcers, the product was used in patients who have neuropathic ulcers. Specifically, the analysis was carried out on patients with grade I or II ulcers on the Wagner scale.

### Specific objectives:

- To test the efficacy of the same galenic formulations claimed for curing venous ulcers in patients with diabetic foot.
- To check if there are significant differences in the drug efficacy in neuropathic ulcers under two different dose application regimens (every 12 hours and every 24 hours).

### Execution:

Having taken into account the difficulty in recruiting patients who complied with the inclusion conditions described above, and since it can be inferred from tests conducted in patients with venous ulcers that the level of efficacy of the galenic gel, cream and aqueous solution formulations in their different concentrations is very similar in all cases, the clinical tests on diabetic foot were performed at only two concentrations: one of them concentrated (10⁻² M) and the other one rather diluted (10⁻⁹ M), both on the gel base. For each them, the base formulation used in the first treated cases of venous ulcers was applied (see section 2), using two different dose regimens (every 12 and 24 hours). The degree of purity of the active ingredient used in this clinical test was 41.5%.

However, these formulations and dose regimens must be considered only by way of example since the effectiveness of the remaining formulations has been demonstrated in patients with venous ulcers.

Eight patients who complied with the criteria described above were evaluated using the following formulations:
- Control group: Two patients to whom the treatment with becaplermin established in section 3.2 above was applied.
   ∘ Patient 1: 5.40 cm² ulcer. Grade I. Application of a dose of the preparation with becaplermin every 24 hours.
   ∘ Patient 2: 5.86 cm² ulcer. Grade I. Application of a dose of the preparation with becaplermin every 24 hours.

The control patients always showed recovery diagnosis equal to or better than the patients to whom the base preparation was applied (Wagner Grade I vs. Wagner Grade I or II).
- 10⁻²M of oleuropein (three patients):
   ∘ Patient 3: 5.58 cm² ulcer. Grade II. Application of a dose of the base preparation every 24 hours.
   ∘ Patient 4: 3.45 cm² ulcer. Grade I. Application of a dose of the base preparation every 24 hours.
   ∘ Patient 5: 2.64 cm² ulcer. Grade II. Application of a dose of the base preparation every 12 hours.
- 10⁻⁹M of oleuropein (three patients):
   ∘ Patient 6: 1.62 cm² ulcer. Grade I. Application of a dose of the base preparation every 24 hours.
   ∘ Patient 7: 7.70 cm² ulcer. Grade I. Application of a dose of the base preparation every 24 hours.
   ∘ Patient 8: 5.56 cm² ulcer. Grade I. Application of a dose of the base preparation every 12 hours.

### Conclusions:

All the patients treated with the preparations containing oleuropein, regardless of the concentration and treatment regimen (number of doses), very significantly reduced the ulcer surface and reduced the classification according to the Wagner-Merrit Classification by about two grades, clearly overcoming the controls in terms of efficacy and without showing very significant differences between the different treatment mechanisms (applications every 12 or 24 hours and gel concentration used).

In four of the six cases treated with oleuropein the ulcer problem which had not experienced any significant improvement (100% healing at the end of treatment) in two or more weeks was completely resolved. The two remaining patients (patients 7 and 8) to whom a lower gel concentration (10⁻⁹ M) was applied achieved healing levels of 86.23% and 99.28%, respectively, versus the average 48.95% ulcerated surface reduction of the control group.

It should be mentioned that the low active ingredient concentrations tested (10⁻⁹ M) had, in any case, a level of efficacy much greater than the control. The ulcer which had a faster progression (mean daily healing of 8.33%) corresponded to patient 6 treated with oleuropein at 10⁻⁹ M in a daily application regimen. However, it was an ulcer with a small diameter (1.62 cm²). See Figure 8.

The secondary objectives (ease of application and safety) were also met: the nurses who applied the composition confirmed that the gel was the easiest to apply (mean score obtained was 8.33 out of 10). Additionally, discomfort or toxicity problems were not detected in patients.

In terms of the more specific objectives mentioned above for the tests on diabetic foot, it can be concluded as proven that the galenic formulations claimed for curing venous ulcers are also effective in patients with diabetic foot, and there are no significant differences in the efficacy of the drug between those cases in which the drug was applied every 12 hours and those in which the drug was applied every 24 hours.

### 6.3. Pressure ulcers (PU)

Once the use of the product of the present invention in its different galenic forms, active ingredient concentrations and dose regimens both in venous ulcers and neuropathic ulcers has been tested with success, it was logical to think that suitably treated pressure ulcers might also show significant improvement with the use of this product.

### Specific objectives:

To test the efficacy of the same galenic formulations claimed for the recovery of venous ulcers and diabetic foot in patients with pressure ulcers. Additionally, the superiority of the product with respect to the controls under different application regimens (doses) was verified.

### Execution:

The tests conducted in patients with pressure ulcers followed a standard different from the previous standards because in order to carry out the tests patients having more than one pressure ulcer showing no surface reduction in at least the last two weeks were selected. Thus, the control would be the patient himself. It was decided that the ulcer with the worst diagnosis (considering the PU scale) and/or largest surface would be treated with oleuropein, while the other ulcer would act as a control following the recovery-related regimens described in section 3. Additionally, in the only patient who had three pressure ulcers in different locations, the differences in the healing rate derived from the use of three different dose regimens of the same product (12 hours, 24 hours, and 48 hours) were analyzed.

The PU Classification mentioned in section 4 (scale 4.3) was used to determine the inclusion or exclusion of the patients in the study. All the patients studied had stage II or III ulcers.

The patients analyzed in this phase of the clinical study are described below:
- Patient 1:
   ∘ Control: 4.96 cm² ulcer in the ischium (Stage III).
   ∘ 10⁻² M gel every 24 hours: 5.32 cm² ulcer in the sacrum (Stage III).
- Patient 2:
   o Control: 8.98 cm² ulcer in the left heel (Stage III).
   o Gel 10⁻⁷ M every 24 hours: 18.03 cm² ulcer in the sacrum (Stage III).
- Patient 3:
   o Control: 8.57 cm² ulcer in the left heel (Stage III).
   o Gel 10⁻⁹ every 24 hours: 11.35 cm² ulcer in the right heel (Stage III and 13 months of duration).
- Patient 4 (dose effect test):
   ∘ Application every 48 hours (10⁻⁴ M gel): 8.37 cm² ulcer in the gluteus (Stage II).
   ∘ Application every 12 hours (10⁻⁴ M gel): 10.56 cm² ulcer in the right heel (Stage II).
   ∘ Application every 24 hours (10⁻⁴ M gel): 1.19 cm² ulcer in the sacrum (Stage II).

### /*/*Conclusions:

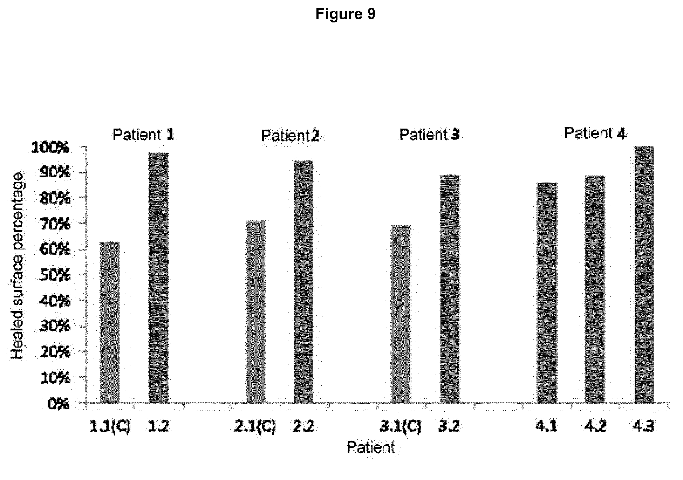

All the dose concentrations and regimens used have an efficacy that exceeds the controls, much greater than the controls, there being no significant differences between them. See Figure 9. The pattern of the most concentrated formulations having results that are very slightly better than the most diluted formulations is again repeated.

As occurred with diabetic foot ulcers, no significant differences were observed between the treatments with applications every 12 hours and those used every 24 hours. (see in Figure 9 the healing percentages of patient 4 under the three application regimens used: 48 hours., 12 hours. and 24 hours.).

It is therefore reasonable to think that in addition to the galenic formulations and concentrations tested by way of example in patients suffering PU, the remaining galenic formulations and concentrations already tested in the previous phases of the present clinical study (venous and neuropathic ulcers) will have a healing capacity that is far superior to that obtained following conventional treatments applied today, regardless of the dose regimen used.

Regarding the main objectives of the study, it should be mentioned that the healing rate of the ulcers treated with oleuropein (objective 5) was far superior in all cases to the ulcers treated with becaplermin (patient 1: 57% faster; patient 2: 34% faster; patient 3: 29% faster), despite the fact that, as mentioned above, the surface of the ulcers treated with oleuropein was much greater than the control ulcers. Additionally, all the ulcers treated with oleuropein which were stage III dropped down to stage I or stage I-II (objective 6).

In terms of the secondary objectives of the study (objectives 7 and 8), all nurses affirmed that the gel was easy to apply (mean score obtained of 8.5 out of 10) and no discomfort or toxicity problems were detected in the patients.

### BIBLIOGRAPHY

1. http://healthychristianliving.com/olive leaf extract.htm
2. Perrinjaquet-Moccetti et al. "Food Supplementation with an Olive (Olea europaea L.) Leaf Extract Reduces Blood Pressure in Borderline Hypertensive Monozygotic Twins", 2008.
3. Somova et al. "Antihypertensive, antiatherosclerotic and antioxidant activity of triterpenoids isolated from Olea europea, subspecies Africana leaves", 2003.
4. Khayyal et al. "Blood pressure lowering effect of an olive leaf extract (Olea euroaea) in L-NAME induced hypertension in rats", 2002.
5. Zarzuelo et al. Vasodilator effect of olive leaf, 1991.
6. DR. Stevenson, L., et al. "Oxygen Radical Absorbance Capacity (ORAC) Report on Olive Leaf Australia's Olive Leaf Extracts, Southern Cross University, 2005.
7. Visioli F, Poli A, and Galli C. "Antioxidant and other biological activities of phenols from olives and olive oil" Med.Res.Rev. 2002, 22: 65-75.
8. Visioli F, Bellosta S, and Galli C, "Oleuropein, the bitter principles of olives, enhances nitric oxide production by mouse macrophages." Life. Sci. 1998, 62: 541-546.
9. Carluccio MA, Siculella L, Ancora MA, Massaro M, Scoditti E, Storelli C, Visioli F, Distante A, De Caterina R. "Olive oil and red wine antioxidant polyphenols inhibit endothelial activation: antiatherogenic properties of Mediterranean diet phytochemicals" Arterioscler Thromb Vasc Biol. 2003; 23:622-629.
10. Owen RW, Giacosa A, Hull WE, Haubner R, Würtele G, Spiegelhalder B, Bartsch H. "Olive oil consumption and health: the possible role of antioxidants." Lancet Oncol. 2000, 1: 107-112.
11. Tripoli E, Giammanco M, Tabacchi G, Di Majo D, Giammanco S, La Guardia M. ""The phenolic composition of olive oil: structure, biological activity, and beneficial effects on human health." Nutr. Res. Rev. 2005, 18:98-112.
12. Fredickson WR, F and S Group, Inc. "Method and composition for Antiviral Therapy with Olive Leaves". US patent 2000; 6:117,884.
13. Andreadou I, Sigala F, Iliodromitis EK, Papaefthimiou M, Sigalas C, Aligiannis N, Savvari P, Gorgoulis V, Papalabros E, Kremastinos DT. "Acute doxorubicin cardiotoxicity is successfully treated with the phytochemical oleuropein through suppression of oxidative and nitrosative stress." J. mol. Cell. Cardio. 2007, 42: 549-558.
14. Andreadou I, Lliodromitis EK, Mikros E, Constantinou M, Agalias A, Magiatis P, Skaltsounia AL, Kamber E, Tsantilil-Kakoulidou A, Kremastinos DT. "The olive constituent oleuropein exhibits anti-ischemic, antioxidative, and hypolipidemic effects in anesthetized rabbits." J. Nut. 2006, 136: 2213-2219.
15. David W. Voigt et al. "Economic Study of Collagen-Glycosaminoglycan Biodegradable Matrix for Chronic Wounds." Wounds 2006, 18(1):1-7.
16. "Scope: Management of type 2 diabetes: prevention and management of foot problems (update)"(PDF). Clinical Guidelines and Evidence Review for Type 2 Diabetes: Prevention and Management of Foot Problems. National Institute for Health and Clinical Excellence. 20 February 2003. http://www.nice.org.uk/nicemedia/pdf/footcare scope.pdf. Retrieved 2007-12-04.
17. Rowe DW et al., "Abnormalities in proliferation and protein synthesis in skin fibroblast cultures from patients with diabetes mellitus". Diabetes 1997, 26:284-290 PMID 849809.
18. Stephen A. Brigido et al., "Use of an Acellular Flowable Dermal Replacement Scaffold on Lower Extremity Sinus Tract Wounds", Foot & Ankle Specialist 2009,2(2):67-72.
19. McLennan S et al, Molecular aspects of wound healing, Primary intention (2006). 14(1):8-13
20. E. Linden et al, Endothelial Dysfunction in Patients with Chronic Kidney Disease Results from Advanced Glycation End Products (AGE)-Mediated Inhibition of Endothelial Nitric Oxide Synthase through RAGE Activation,Clin. J. Am. Soc. Nephrol (2008).3(3): 691 - 698 PMID 18256374
21. Galkowska H et.al, Chemokines, cytokines and growth factors in keratinocytes and dermal endothelial cells in the margin if chronic diabetic foot ulcers, Wound Repair Regen (2006). 14:558-565 PMID 17014667
22. Neil Bennett et al, Growth factors and wound healing: Part II. Role in normal and chronic wound healing, Am J Surg (1993). 166: 74-81 PMID 8392302
23. Debbie Sharman, Moist wound healing: a review of evidence, application and outcome, The Diabetic Foot (2003). 6(3): 112-120
24. lakovos N Nomikos et al, Protective and Damaging Aspects of Healing: A Review, Wounds (2006). 18 (7): 177-185.
25. Harold Brem, Marjana Tomic-Canic. "Cellular and Molecular basis of wound healing in diabetes". JCI 2007,117(5):1219-1222. PMID 17476353.
26. Galkowska H.et al. "Chemokines, cytokines and growth factors in keratinocytes and dermal endothelial cells in the margin of chronic diabetic foot ulcers". Wound Repair Regen 2006,14:558-565 PMID 17014667.
27. Spencer S; Spencer, Sue A. "Pressure relieving interventions for preventing and treating diabetic foot ulcers". 2000, Cochrane Database System Rev.3:CD002302.doi:10.1002/14651858.CD002302. PMID 10908550.
28. Schäffer M, Bongartz M, Fisher S, Proksch B, Viebahn R. "Nitric oxide restores impaired healing in normoglycemiaemic diabetic rats". J. Wound Care (July 2007) 16(7):311-6. PMID 17708383.
29. Zamboni WA, Wong HP, Stephenson LL, Pfeifer MA (September 1997). "Evaluation of hyperbaric oxygen for diabetic wounds: a prospective study". Undersea Hyperb Med 24(3):175-9. PMID 9308140.
30. Kranke P, Benett M, Roeckl-Wiedmann I, Debus S (2004) "Hyperbaric oxygen therapy for chronic wounds" Cochrane Database Sys Rev (2): CD004123. PMID 15106239.
31. Snyder RJ (2005). "Treatment of nonhealing ulcers with allografts". Clin.Dermatol. 23(4):388-95. PMID 16023934.
32. Gardner SE, Frantz RA, Doebbeling BN. "The validity of the clinical sings and symptoms used to identify localized chronic wound infection". Wound Repair Regen. 2001; 9 (3): 178-86.
33. O'Meara S, Al Kurdi D, Ovington LG. "Antibiotics and antiseptics for venous ulcers" (review). The Cocrhane Library 2008. CD003557.
34. Gray D, Cooper P. "Nutrition and wound healing: what is the link?" J Wound Care. 2001; 10(3):86-9.
35. Jessup RL. "What is the best method for assessing the rate of wound healing? A comparison of 3 mathematical formulas". Adv Skin Wound Care. 2006; 19:138-47.
36. Marinello J. "Metodos de evaluación de las úlceras. Criterios predictivos de curación". In: Marinello Roura J, editor. Úlceras de la extremidad inferior. Barcelona: Glosa; 2005. p. 275-85.
37. Buntinx F, Beckers H, De Keyser G, Flour M, Nissen G, et al. "Inter-observer variation in the assessment of skin ulceration" .J Wound Care. 1996; 5(4):166-70.
38. Palomar F, Fornés B, Tornero A, Muñoz A. "Escala de Valoración FED-PALLA de la piel perilesional". Enfermería Dermatológica 2007; 1:36-8
39. Wagner FW. "The dysvascular foot: a system for diagnosis and treatment". Foot Ankle. 1981; 2:64-122.
40. European Pressure Ulcer Advisory Panel (EPUAP). "Guidelines for Pressure Ulcer Treatment". [Internet]. Available at http://www.epuap.org/index.html. Translation:http://www.gneaupp.org/documentos/epuap/directrices.txt)

## Claims

1. A pharmaceutical composition containing oleuropein as the only active ingredient for use in the treatment of healing wounds and ulcers in persons of advanced age or the elderly and/or diabetics selected from the group consisting of: diabetic foot, pressure ulcer and venous ulcer.

2. A pharmaceutical composition containing oleuropein as the only active ingredient for use according to claim 1, wherein the pharmaceutical composition comprises in addition to the active ingredient oleuropein, a gelling agent, a preservative, a solvent, an alkalizing agent and optionally an antioxidant.

3. A pharmaceutical composition containing oleuropein as the only active ingredient for use according to any of the preceding claims, wherein the pharmaceutical composition is presented in a gel form and additionally comprises a pharmaceutically acceptable carrier.

4. A pharmaceutical composition containing oleuropein as the only active ingredient for use according to any of claims 1 to 2, wherein the pharmaceutical composition is presented in a cream form and additionally comprises a pharmaceutically acceptable carrier.

5. A pharmaceutical composition containing oleuropein as the only active ingredient for use according to any of claims 1 to 4, wherein the oleuropein is present in the pharmaceutical composition at a concentration of 10⁻¹ M to 10⁻¹¹ M.

6. A pharmaceutical composition containing oleuropein as the only active ingredient for use according to claim 5, wherein the oleuropein is present in the pharmaceutical composition at a concentration of 10⁻² to 10⁻⁹M.

7. A pharmaceutical composition containing oleuropein as the only active ingredient for use according to any of claims 1 to 6, wherein the treatment of healing wounds and ulcers in persons of advanced age or the elderly and/or diabetics is diabetic foot.

8. A pharmaceutical composition containing oleuropein as the only active ingredient for use according to any of claims 1 to 6, wherein the treatment of healing wounds and ulcers in persons of advanced age or the elderly and/or diabetics is venous ulcers.

9. A pharmaceutical composition containing oleuropein as the only active ingredient for use according to any of claims 1 to 6, wherein the treatment of healing wounds and ulcers in persons of advanced age or the elderly and/or diabetics is pressure ulcers.

10. A pharmaceutical composition containing oleuropein as the only active ingredient for use according to any of claims 1 to 9, wherein the treatment comprises an application regimen at 12 hours, 24 hours or 48 hours.

## Patentansprüche

1. Pharmazeutische Zusammensetzung mit Oleuropein als einzigem Wirkstoff zur Verwendung in der Heilbehandlung von Wunden und Geschwüren bei Personen fortgeschrittenen Alters oder älteren Menschen und/oder Diabetikern, ausgewählt aus der Gruppe, bestehend aus: diabetischem Fuß, Druckgeschwür und venösem Geschwür.

2. Pharmazeutische Zusammensetzung, welche Oleuropein als einzigen Wirkstoff enthält, zur Verwendung nach Anspruch 1, wobei die pharmazeutische Zusammensetzung zusätzlich zu dem Wirkstoff Oleuropein ein Geliermittel, ein Konservierungsmittel, ein Lösungsmittel, einen alkalisierenden Wirkstoff und wahlweise ein Antioxidationsmittel umfasst.

3. Pharmazeutische Zusammensetzung, welche Oleuropein als einzigen Wirkstoff enthält, zur Verwendung nach einem der vorangehenden Ansprüche, wobei die pharmazeutische Zusammensetzung in einer Gelform bereitgestellt wird und zusätzlich einen pharmazeutisch verträglichen Träger umfasst.

4. Pharmazeutische Zusammensetzung, welche Oleuropein als einzigen Wirkstoff enthält, zur Verwendung nach einem der Ansprüche 1 bis 2, wobei die pharmazeutische Zusammensetzung in einer Cremeform bereitgestellt wird und zusätzlich einen pharmazeutisch akzeptablen Träger umfasst.

5. Pharmazeutische Zusammensetzung, welche Oleuropein als einzigen Wirkstoff enthält, zur Verwendung nach einem der Ansprüche 1 bis 4, wobei das Oleuropein in der pharmazeutischen Zusammensetzung in einer Konzentration von 10⁻¹ M bis 10⁻¹¹ M vorhanden ist.

6. Pharmazeutische Zusammensetzung, welche Oleuropein als einzigen Wirkstoff enthält, zur Verwendung nach Anspruch 5, wobei das Oleuropein in der pharmazeutischen Zusammensetzung in einer Konzentration von 10⁻² bis 10⁻⁹ vorhanden ist.

7. Pharmazeutische Zusammensetzung, welche Oleuropein als einzigen Wirkstoff enthält, zur Verwendung nach einem der Ansprüche 1 bis 6, wobei die Heilbehandlung von Wunden und Geschwüren bei Personen fortgeschrittenen Alters oder älteren Menschen und/oder Diabetikern der diabetische Fuß ist.

8. Pharmazeutische Zusammensetzung, welche Oleuropein als einzigen Wirkstoff enthält, zur Verwendung nach einem der Ansprüche 1 bis 6, wobei die Heilbehandlung von Wunden und Geschwüren bei Personen fortgeschrittenen Alters oder älteren Menschen und/oder Diabetikern venöse Druckgeschwüre sind.

9. Pharmazeutische Zusammensetzung, welche Oleuropein als einzigen Wirkstoff enthält, zur Verwendung nach einem der Ansprüche 1 bis 6, wobei die Heilbehandlung von Wunden und Geschwüren bei Personen fortgeschrittenen Alters oder älteren Menschen und/oder Diabetikern Druckgeschwüre sind.

10. Pharmazeutische Zusammensetzung, welche Oleuropein als einzigen Wirkstoff enthält, zur Verwendung nach einem der Ansprüche 1 bis 9, wobei die Behandlung einen Anwendungsplan über 12 Stunden, 24 Stunden oder 48 Stunden umfasst.

## Revendications

1. Composition pharmaceutique contenant de l'oleuropéine comme seul ingrédient actif pour son utilisation dans le traitement de cicatrisation de plaies et d'ulcères chez les personnes d'âge mûr ou les personnes âgées et/ou diabétiques choisies dans le groupe consistant en : pied diabétique, ulcère de pression et ulcère veineux.

2. Composition pharmaceutique contenant de l'oleuropéine comme seul ingrédient actif pour son utilisation selon la revendication 1, dans laquelle la composition pharmaceutique comprend outre l'ingrédient actif l'oleuropéine, un agent gélifiant, un conservateur, un solvant, un agent alcalinisant et optionnellement un antioxydant.

3. Composition pharmaceutique contenant de l'oleuropéine comme seul ingrédient actif pour son utilisation selon l'une quelconque des revendications précédentes, dans laquelle la composition pharmaceutique est présentée sous forme de gel et comprend en outre un véhicule pharmaceutiquement acceptable.

4. Composition pharmaceutique contenant de l'oleuropéine comme seul ingrédient actif pour son utilisation selon l'une quelconque des revendications 1 à 2, dans laquelle la composition pharmaceutique est présentée sous forme de crème et comprend en outre un véhicule pharmaceutiquement acceptable.

5. Composition pharmaceutique contenant de l'oleuropéine comme seul ingrédient actif pour son utilisation selon l'une quelconque des revendications 1 à 4, dans laquelle l'oleuropéine est présente dans la composition pharmaceutique à une concentration de 10⁻¹ M à 10⁻¹¹ M.

6. Composition pharmaceutique contenant de l'oleuropéine comme seul ingrédient actif pour son utilisation selon la revendication 5, dans laquelle l'oleuropéine est présente dans la composition pharmaceutique à une concentration de 10⁻² à 10⁻⁹ M.

7. Composition pharmaceutique contenant de l'oleuropéine comme seul ingrédient actif pour son utilisation selon l'une quelconque des revendications 1 à 6, dans laquelle le traitement de cicatrisation de plaies et d'ulcères chez les personnes d'âge mûr ou les personnes âgées et/ou diabétiques est le pied diabétique.

8. Composition pharmaceutique contenant de l'oleuropéine comme seul ingrédient actif pour son utilisation selon l'une quelconque des revendications 1 à 6, dans laquelle le traitement de cicatrisation de plaies et d'ulcères chez les personnes d'âge mûr ou les personnes âgées et/ou diabétiques est les ulcères veineux.

9. Composition pharmaceutique contenant de l'oleuropéine comme seul ingrédient actif pour son utilisation selon l'une quelconque des revendications 1 à 6, dans laquelle le traitement de cicatrisation de plaies et d'ulcères chez les personnes d'âge mûr ou les personnes âgées et/ou diabétiques est les ulcères de pression.

10. Composition pharmaceutique contenant de l'oleuropéine comme seul ingrédient actif pour son utilisation selon l'une quelconque des revendications 1 à 9, dans laquelle le traitement comprend un régime d'application à 12 heures, 24 heures ou 48 heures.
